# EUROPÄISCHE PATENTSCHRIFT

(11) **EP 2 203 448 B1**
(45) Veröffentlichungstag und Bekanntmachung des Hinweises auf die Patenterteilung: **22.06.2011**
(21) Anmeldenummer: 08785816.3
(22) Anmeldetag: 04.09.2008
(51) Int. Cl.: C07D 417/12, C07D 513/04, A61K 31/5415, A61P 3/10

(54) **PHENOTHIAZIN-DERIVATE MIT DOPPELBINDUNG, VERFAHREN ZU IHRER HERSTELLUNG UND IHRE VERWENDUNG ALS ARZNEIMITTEL**
PHENOTHIAZINE DERIVATIVE HAVING A DOUBLE BOND, METHOD FOR THE PRODUCTION THEREOF, AND USE THEREOF AS A PHARMACEUTICAL
DÉRIVÉS DE PHÉNOTHIAZINE À LIAISON DOUBLE, PROCÉDÉ POUR LES PRODUIRE ET LEUR UTILISATION COMME MÉDICAMENTS

(30) Priorität: 21.09.2007 EP 07291133
(43) Veröffentlichungstag der Anmeldung: 07.07.2010
(73) Patentinhaber: Sanofi-Aventis, 75013 Paris (FR)
(72) Erfinder: KEIL, Stefanie, 65926 Frankfurt am Main (DE); DEFOSSA, Elisabeth, 65926 Frankfurt am Main (DE); SCHMOLL, Dieter, 65926 Frankfurt am Main (DE); DIETRICH, Axel, 65926 Frankfurt am Main (DE); KUHLMANN, Johanna, 65926 Frankfurt am Main (DE); ENGEL, Karl-Christian, 65926 Frankfurt am Main (DE)
(74) Vertreter: Fischer, Hans-Jürgen
(86) Internationale Anmeldenummer: PCT/EP2008/007219
(87) Internationale Veröffentlichungsnummer: WO 2009/039944

(56) Entgegenhaltungen:
- WO-A-01/44216
- WO-A-02/062772
- WO-A-2005/095417

## Beschreibung

Die Erfindung betrifft substituierte Phenothiazine mit Doppelbindung sowie deren physiologisch verträgliche Salze.

Es sind bereits Penothiazin Derivate wie zum Beispiel Chlorpromazin (3-(2-Chlor-4a,10a-dihydro-10*H-*phenothiazin-10-yl)-*N,N*-dimethylpropan-1-amin) als Neuroleptika bekannt. WO 01/44216 beschreibt strukturaähnliche Verbindungen.

Der Erfindung lag die Aufgabe zugrunde, Verbindungen zur Behandlung von Diabetes zu entwickeln. Diese Verbindungen sollen inbesondere den Blutzuckerspiegel absenken.

Die Erfindung betrifft daher Verbindungen der Formel I, 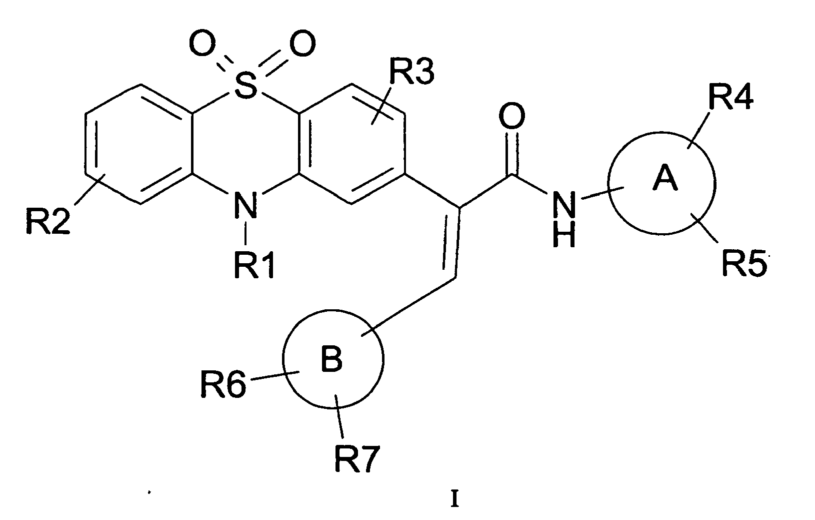 worin bedeuten
- R1: H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl
- R2, R3: unabhängig voneinander H, F, Cl, Br, CN, NO₂, (C₀-C₆)-Alkylen-COO-(C₀-C₆)- Alkyl, (C₀-C₆)-Alkylen-O-(C₀-C₆)-Alkyl, (C₁-C₆)-Alkyl, (C₀-C₆)-Alkylen-CO- (C₁-C₆)-Alkyl, (C₀-C₆)-Alkylen-Phenyl, SCF₃, SF₅, SCH₃;
- R4, R5: unabhängig voneinander H, F, Cl, Br, CN, SCN, NO₂, (C₀-C₆)-Alkylen-COO- (C₀-C₆)-Alkyl, -CO-COO-(C₀-C₆)-Alkyl, (C₀-C₆)-Alkylen-O-(C₀-C₆)-Alkyl, (C₁- C₆)-Alkyl, (C₀-C₆)-Alkylen-CO-(C₁-C₆)-Alkyl, (C₁-C₆)-Alkylen-CONH(C₀-C₆)- Alkyl, (C₀-C₆)-Alkylen-CON[(Co-C₆)-Alkyl]₂, (C₀-C₆)-Alkylen-NH(C₀-C₆)- Alkyl, (C₀-C₆)-Alkylen-NH-COO-(C₀-C₆)-Alkyl, (C₀-C₆)-Alkylen-CON[(C₀-C₆)- Alkyl]-O-(C₀-C₆)-Alkyl, (C₀-C₆)-Alkylen-N[(C₀-C₆)-Alkyl]₂, (C₀-C₆)-Alkylen- Aryl, SF₅, (C₀-C₆)-Alkyl-S(O)ₓ(C₁-C₆)-Alkyl, S(O)ₓ(C₁-C₆)-Alkylen-COO-(C₀- C₆)-Alkyl, S(O)ₓ(C₂-C₆)-Alkylen-O-(C₀-C₆)-Alkyl, -SO₂-NH-(C₀-C₆)-Alkyl, -SO₂-N-[(C₀-C₆)-Alkyl]₂, S(O)ₓ(C₀-C₆)-Alkylen-Heterocyclus, S(O)ₓ(C₁-C₆)- Alkylen-CO-Heterocyclus, -NH-SO₂-(C₁-C₆)-Alkyl, (C₀-C₆)-Alkylen-Cycloalkyl, (C₀-C₆)-Alkylen-Heterocyclus, (C₀-C₆)-Alkylen-Aryl;
- R6, R7: unabhängig voneinander H, F, Cl, Br, CN, NO₂, =O, =S, =N-O-(C₀-C₆)-Alkyl, (C₀-C₆)-Alkylen-COO-(C₀-C₆)-Alkyl, (C₀-C₆)-Alkylen-O-(C₀-C₆)-Alkyl, (C₀-C₆)- Alkylen-O-CO-(C₁-C₆)-Alkyl, (C₁-C₆)-Alkyl, (C₀-C₆)-Alkylen-CO-(C₁-C₆)- Alkyl, (C₀-C₆)-Alkylen-Aryl, SF₅, S(O)ₓ-(C₁-C₆)-Alkyl;
- A: 5 bis 10 gliedriger Heterocyclus, wobei der Heterocyclus mit einem weiteren 5 bis 10 gliedrigen Ring anneliert sein kann;
- B: 4 bis 8 gliedriger Cycloalkylring, ein 4 bis 10 gliedriger Heterocyclus oder ein 6 bis 10 gliedriger Arylring;
sowie deren physiologisch verträgliche Salze.

Bevorzugt sind Verbindungen der Formel I, worin ein oder mehrere Reste folgende Bedeutung haben:
- R1: H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl
- R2, R3: unabhängig voneinander H, F, Cl, Br, CN, NO₂, (C₀-C₆)-Alkylen-COO-(C₀-C₆)- Alkyl, (C₀-C₆)-Alkylen-O-(C₀-C₆)-Alkyl, (C₁-C₆)-Alkyl, (C₀-C6)-Alkylen-CO- (C₁-C₆)-Alkyl, (C₀-C₆)-Alkylen-Phenyl, SCF₃, SF₅, SCH₃;
- R4, R5: unabhängig voneinander H, F, Cl, Br, CN, SCN, NO₂, (C₀-C₆)-Alkylen-COO- (C₀-C6)-Alkyl, -CO-COO-(C₀-C₆)-Alkyl, (C₀-C₆)-Alkylen-O-(C₀-C₆)-Alkyl, (C₁- C₆)-Alkyl, (C₀-C6)-Alkylen-CO-(C₁-C₆)-Alkyl, (C₀-C₆)-Alkylen-CONH(C₀-C₆)- Alkyl, (C₀-C₆)-Alkylen-CON[(C₀-C₆)-Alkyl]₂, (C₀-C₆)-Alkylen-NH(C₀-C₆)- Alkyl, (C₀-C₆)-Alkylen-NH-COO-(C₀-C₆)-Alkyl, (C₀-C₆)-Alkylen-CON[(C₀-C₆)- Alkyl]-O-(C₀-C₆)-Alkyl, (C₀-C₆)-Alkylen-N[(C₀-C₆)-Alkyl]₂, (C₀-C₆)-Alkylen- Aryl, SF₅, (C₀-C₆)-Alkyl-S(O)ₓ(C₁-C₆)-Alkyl, S(O)ₓ(C₁-C₆)-Alkylen-COO-(C₀- C₆)-Alkyl, S(O)ₓ(C₂-C₆)-Alkylen-O-(C₀-C₆)-Alkyl, -SO₂-NH-(C₀-C₆)-Alkyl, -SO₂-N-[(C₀-C₆)-Alkyl]₂, S(O)ₓ(C₀-C₆)-Alkylen-Heterocyclus, S(O)ₓ(C₁-C₆)- Alkylen-CO-Heterocyclus, -NH-SO₂-(C₁-C₆)-Alkyl, (C₀-C₆)-Alkylen-Cycloalkyl, (C₀-C₆)-Alkylen-Heterocyclus, (C₀-C₆)-Alkylen-Aryl;
- R6, R7: unabhängig voneinander H, F, Cl, Br, CN, NO₂, O, =S, =N-O-(C₀-C₆)-Alkyl, (C₀-C₆)-Alkylen-COO-(C₀-C₆)-Alkyl, (C₀-C₆)-Alkylen-O-(C₀-C₆)-Alkyl, (C₀-C₆)- Alkylen-O-CO-(C₁-C₆)-Alkyl, (C₁-C₆)-Alkyl, (C₀-C₆)-Alkylen-CO-(C₁-C₆)- Alkyl, (C₀-C₆)-Alkylen-Aryl, SF₅, S(O)ₓ-(C₁-C₆)-Alkyl;
- A: 5 bis 10 gliedriger Heterocyclus, wobei der Heterocyclus mit einem weiteren 5 bis 10 gliedrigen Ring anneliert sein kann;
- B: 4 bis 8 gliedriger Cycloalkylring;
sowie deren physiologisch verträgliche Salze.

Besonders bevorzugt sind Verbindungen der Formel I, worin ein oder mehrere Reste folgende Bedeutung haben:
- R1: H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl
- R2, R3: H;
- R4: (C₁-C₆)-Alkyl, O-(C₁-C₆)-Alkyl, (C₀-C₆)-Alkylen-Aryl;
- R5: H;
- R6, R7: unabhängig voneinander H, F, Cl, Br, CN, NO₂, =O, =S, =N-O-(C₀-C₆)-Alkyl, (C₀-C₆)-Alkylen-COO-(C₀-C₆)-Alkyl, (C₀-C₆)-Alkylen-O-(C₀-C₆)-Alkyl, (C₀-C₆)- Alkylen-O-CO-(C₁-C₆)-Alkyl, (C₁-C₆)-Alkyl, (C₀-C₆)-Alkylen-CO-(C₁-C₆)- Alkyl, (C₀-C₆)-Alkylen-Aryl, SF₅, S(O)ₓ-(C₁-C₆)-Alkyl;
- A: 5 bis 10 gliedriger Heterocyclus, wobei der Heterocyclus mit einem weiteren 5 bis 10 gliedrigen Ring anneliert sein kann;
- B: 4 bis 8 gliedriger Cycloalkylring;
sowie deren physiologisch verträgliche Salze.

In einer Ausführungsform sind Verbindungen der Formel I bevorzugt, in denen R1 gleich H ist.

In einer Ausführungsform sind Verbindungen der Formel I bevorzugt, in denen R1 gleich Methyl ist.

In einer Ausführungsform sind Verbindungen der Formel I bevorzugt, in denen A gleich Pyrazol-3-yl ist.

In einer Ausführungsform sind Verbindungen der Formel I bevorzugt, in denen A gleich Thiazolo[5,4-b]pyridin-2-yl ist.

In einer Ausführungsform sind Verbindungen der Formel I bevorzugt, in denen B gleich Cyclopentyl ist.

In einer Ausführungsform sind Verbindungen der Formel I bevorzugt, in denen B gleich Cyclohexyl ist.

In einer Ausführungsform sind Verbindungen der Formel I bevorzugt, in denen R6 gleich H ist.

In einer Ausführungsform sind Verbindungen der Formel I bevorzugt, in denen R6 gleich =O ist.

In einer Ausführungsform sind Verbindungen der Formel I bevorzugt, in denen R7 gleich H ist.

In einer Ausführungsform sind Verbindungen der Formel I bevorzugt, in denen R4 gleich -O-(C₁-C₆)-Alkyl ist.

In einer Ausführungsform sind Verbindungen der Formel I bevorzugt, in denen R4 gleich C₁-C₆)-Alkyl ist.

In einer Ausführungsform sind Verbindungen der Formel I bevorzugt, in denen R4 gleich Benzyl ist.

Die Erfindung bezieht sich auf Verbindungen der Formel I, in Form ihrer Racemate, racemischen Mischungen und reinen Enantiomere sowie auf ihre Diastereomere und Mischungen davon.

Können Reste oder Substituenten mehrfach in den Verbindungen der Formel I auftreten, so können sie alle unabhängig voneinander die angegebenen Bedeutungen haben und gleich oder verschieden sein.

Unter der Defintion (C₀-C₆)-Alkylen- wird verstanden, dass entweder eine Bindung oder eine (C₁-C₆)-Alkylen Gruppe vorhanden sein kann.

Unter der Defintion -(C₀-C₆)-Alkyl wird verstanden, dass entweder ein Wasserstoff oder eine (C₁-C₆)-Alkyl Gruppe vorhanden sein kann.

Unter "Anellierung" oder "anelliert" wird verstanden, dass ein weiteres Ringssystem ankondensiert ist. Das weitere ankondensierte Ringssystem kann aromatisch oder nichtaromatisch und carbocyclisch oder heterocyclisch sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamolsalz (2-Amino-2-hydroxymethyl-1,3-propandiol), Diethanolaminsalz, Lysinsalz oder Ethylendiaminsalz.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Ein weiterer Aspekt der Erfindung sind die physiologisch funktionellen Derivate der Verbindungen der Formel I. Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Unter einem Alkylrest wird eine geradkettige oder verzweigte Kohlenwasserstoffkette mit einem oder mehreren Kohlenstoffen verstanden, wie z.B. Methyl, Ethyl, iso-Propyl, tert.-Butyl, Hexyl.

Die Alkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂ wobei n = 0 - 6 sein kann und der Arylrest oder heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₂-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterocyclus, N(C₁-C₆)-Alkyl-COO-Aryl, N[(C₁-C₆)-Alkyl]-COO-Heterocyclus, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterocyclus, N[(C₁-C₆)-Alkyl]-CO-N-[(C₁-C₆)-Alkyl]₂, N[(C₁-C₆)-Alkyl]-CO-N[(C₁-C₆)-Alkyl)]-Aryl, N[(C₁-C₆)-Alkyl]-CO-N[(C₁-C₆)-Alkyl]-Heterocyclus, N[(C₁-C₆)-Alkyl]-CO-N-(Aryl)₂, N[(C₁-C₆)-Alkyl]-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl, N(Aryl)-CO-NH-Aryl, N(Heterocyclus)-CO-NH-Aryl, N(Aryl)-CO-N-[(C₁-C₆)-Alkyl]₂, N(Heterocyclus)-CO-N-[(C₁-C₆)-Alkyl]₂, N(Aryl)-CO-N[(C₁-C₆)-Alkyl]-Aryl, N(Heterocyclus)-CO-N[(C₁-C₆)-Alkyl]-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N[(C₁-C₆)-Alkyl]₂, SO₂-CH₃, COOH, COO-(C₁-C₆)-Alkyl oder CONH₂.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl.

Die Alkenylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂ wobei n = 0 - 6 sein kann und der Arylrest oder heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₂-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterocyclus, N(C₁-C₆)-Alkyl-COO-Aryl, N[(C₁-C₆)-Alkyl]-COO-Heterocyclus, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterocyclus, N[(C₁-C₆)-Alkyl]-CO-N-[(C₁-C₆)-Alkyl]₂, N[(C₁-C₆)-Alkyl]-CO-N[(C₁-C₆)-Alkyl)]-Aryl, N[(C₁-C₆)-Alkyl]-CO-N[(C₁-C₆)-Alkyl]-Heterocyclus, N[(C₁-C₆)-Alkyl]-CO-N-(Aryl)₂, N[(C₁-C₆)-Alkyl]-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl, N(Aryl)-CO-NH-Aryl, N(Heterocyclus)-CO-NH-Aryl, N(Aryl)-CO-N-[(C₁-C₆)-Alkyl]₂, N(Heterocyclus)-CO-N-[(C₁-C₆)-Alkyl]₂, N(Aryl)-CO-N[(C₁-C₆)-Alkyl]-Aryl, N(Heterocyclus)-CO-N[(C₁-C₆)-Alkyl]-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N[(C₁-C₆)-Alkyl]₂, SO₂-CH₃, COOH, COO-(C₁-C₆)-Alkyl oder CONH₂.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Hexinyl.

Die Alkinylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus;
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0-6 sein kann und der Arylrest oder heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₂-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterocyclus, N(C₁-C₆)-Alkyl-COO-Aryl, N[(C₁-C₆)-Alkyl]-COO-Heterocyclus, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterocyclus, N[(C₁-C₆)-Alkyl]-CO-N-[(C₁-C₆)-Alkyl]₂, N[(C₁-C₆)-Alkyl]-CO-N[(C₁-C₆)-Alkyl)]-Aryl, N[(C₁-C₆)-Alkyl]-CO-N[(C₁-C₆)-Alkyl]-Heterocyclus, N[(C₁-C₆)-Alkyl]-CO-N-(Aryl)₂, N[(C₁-C₆)-Alkyl]-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl, N(Aryl)-CO-NH-Aryl, N(Heterocyclus)-CO-NH-Aryl, N(Aryl)-CO-N-[(C₁-C₆)-Alkyl]₂, N(Heterocyclus)-CO-N-[(C₁-C₆)-Alkyl]₂, N(Aryl)-CO-N[(C₁-C₆)-Alkyl]-Aryl, N(Heterocyclus)-CO-N[(C₁-C₆)-Alkyl]-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N[(C₁-C₆)-Alkyl]₂, SO₂-CH₃, COOH, COO-(C₁-C₆)-Alkyl oder CONH₂.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0-6 sein kann und der Arylrest oder heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₂-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterocyclus, N(C₁-C₆)-Alkyl-COO-Aryl, N[(C₁-C₆)-Alkyl]-COO-Heterocyclus, N[(C₁-C₆)-Alkyl]-CO-NH-(C₁-C₆)-Alkyl, N[(C₁-C₆)-Alkyl]-CO-NH-Aryl, N[(C₁-C₆)-Alkyl]-CO-NH-Heterocyclus, N[(C₁-C₆)-Alkyl]-CO-N-[(C,-C₆)-Alkyl]₂, N[(C₁-C₆)-Alkyl]-CO-N[(C₁-C₆)-Alkyl)]-Aryl, N[(C₁-C₆)-Alkyl]-CO-N[(C₁-C₆)-Alkyl]-Heterocyclus, N[(C₁-C₆)-Alkyl]-CO-N-(Aryl)₂, N[(C₁-C₆)-Alkyl]-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl, N(Aryl)-CO-NH-Aryl, N(Heterocyclus)-CO-NH-Aryl, N(Aryl)-CO-N-[(C₁-C₆)-Alkyl]₂, N(Heterocyclus)-CO-N-[(C₁-C₆)-Alkyl]₂, N(Aryl)-CO-N[(C₁-C₆)-Alkyl]-Aryl, N(Heterocyclus)-CO-N[(C₁-C₆)-Alkyl]-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N[(C₁-C₆)-Alkyl]₂, SO₂-CH₃, COOH, COO-(C₁-C₆)-Alkyl oder CONH₂.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.

Die Cycloalkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C6)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₂-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterocyclus, N(C₁-C₆)-Alkyl-COO-Aryl, N[(C₁-C₆)-Alkyl]-COO-Heterocyclus, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterocyclus, N[(C₁-C₆)-Alkyl]-CO-N-[(C₁-C₆)-Alkyl]₂, N[(C₁-C₆)-Alkyl]-CO-N[(C₁-C₆)-Alkyl)]-Aryl, N[(C₁-C₆)-Alkyl]-CO-N[(C₁-C₆)-Alkyl]-Heterocyclus, N[(C₁-C₆)-Alkyl]-CO-N-(Aryl)₂, N[(C₁-C₆)-Alkyl]-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl, N(Aryl)-CO-NH-Aryl, N(Heterocyclus)-CO-NH-Aryl, N(Aryl)-CO-N-[(C₁-C₆)-Alkyl]₂, N(Heterocyclus)-CO-N-[(C₁-C₆)-Alkyl]₂, N(Aryl)-CO-N[(C₁-C₆)-Alkyl]-Aryl, N(Heterocyclus)-CO-N[(C₁-C₆)-Alkyl]-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N[(C₁-C₆)-Alkyl]₂, SO₂-CH₃, COOH, COO-(C₁-C₆)-Alkyl oder CONH₂.

Unter Heterocyclus bzw. heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterocyclische Rest mit einem weiteren Ringsystem anelliert ist..

Geeignete "Heterocyclen" bzw. "heterocyclische Reste" sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, 5,6-Dihydro-4H-cyclopentathiazol-2-yl, 4,5-Dihydro-thiazol-2-yl, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, 4,5,6,7-Tetrahydro-benzooxazol-2-yl, 4,5,6,7-Tetrahydro-benzothiazol-2-yl, 4,5,6,7-Tetrahydro-benzoimidazol-2-yl, 4,5,6,7-Tetrahydro-pyrazolo[1,5-a]pyridin-2-yl, Tetrahydrofuranyl, Tetrahydropyranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazinyl, Triazolyl, Tetrazolyl, Thiazolo[4,5-b]pyridinyl, Thieno[2,3-d]thiazol-2-yl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-pyridyl.

Umfasst sind weiterhin ein oder mehrfach benzoannelierte Derivate dieser Heterocyclen.

Die Heterocyclen bzw. heterocyclischen Reste können ein oder mehrfach mit geeigneten Gruppen substituiert sein, wie z.B: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁,-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂),-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ(Heterocyclus)₂ wobei n = 0 - 6 sein kann und der Arylrest oder heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₂-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N[(C₁-C₆)-Alkyl]-CO-(C₁-C₆)-Alkyl, N[(C₁-C₆)-Alkyl]-COO-(C₁-C₆)-Alkyl, N[(C₁-C6)-Alkyl]-CO-Aryl, N[(C₁-C₆)-Alkyl]-CO-Heterocyclus, N(C₁-C₆)-Alkyl-COO-Aryl, N[(C₁-C₆)-Alkyl]-COO-Heterocyclus, N[(C₁-C₆)-Alkyl]-CO-NH-(C₁-C₆)-Alkyl), N[(C₁-C₆)-Alkyl]-CO-NH-Aryl, N[(C₁-C6)₋Alkyl]-CO-NH-Heterocyclus, N[(C₁-C₆)-Alkyl]-CO-N-[(C₁-C₆)-Alkyl]₂, N[(C₁-C6)-Alkyl]-CO-N[(C₁-C₆)-Alkyl)]-Aryl, N[(C₁-C₆)-Alkyl]-CO-N[(C₁-C₆)-Alkyl]-Heterocyclus, N[(C_{I}-C₆)-Alkyl]-CO-N-(Aryl)₂, N[(C₁-C₆)-Alkyl]-CO-N-(Heterocyclus)₂, N(Aryl)-CO-(C₁-C₆)-Alkyl, N(Heterocyclus)-CO-(C₁C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl, N(Aryl)-CO-NH-Aryl, N(Heterocyclus)-CO-NH-Aryl, N(Aryl)-CO-N-[(C₁-C₆)-Alkyl]₂, N(Heterocyclus)-CO-N-[(C₁-C₆)-Alkyl]₂, N(Aryl)-CO-N[(C₁-C₆)-Alkyl]-Aryl, N(Heterocyclus)-CO-N[(C₁-C₆)-Alkyl]-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N[(C₁-C₆)-Alkyl]₂, SPO₂-CH₃, COOH, COO-(C₁-C₆)-Alkyl oder CONH₂.

Verbindungen der Formel I aktivieren den Glucosestoffwechsel in Glucokinase-exprimierenden Zellen. Sie sind daher gut zur Behandlung und Verhinderung erhöhter Blutzuckerspiegel, Obesitas und des metabolischen Syndroms geeignet (Sagen et al. Diabetes 55, 1713-1722, Levin et al. Diabetes (2006), S122-S130, Matschinsky et al (2006) 55, 1-12).

Aufgrund der Aktivierung von Glucokinase können die Verbindungen der Formel I auch zur Behandlung bzw. Prävention weiterer Krankheiten und Leiden in einem Säugetier, vorzugsweise einem Menschen, geeignet sein, die durch erhöhten Blutzuckerspiegel, Übergewicht oder durch verringerte Aktivität von Glucokinase hervorgerufen werden.

Die Verbindungen der vorliegenden Erfindung eignen sich insbesondere zur Behandlung und/oder Prävention von:
1. - Glucoseverwertungsstörungen und Störungen des Fettsäurestoffwechsels - Störungen, bei denen Insulinresistenz eine Rolle spielt
   Diabetes mellitus, insbesondere Typ-2-Diabetes, einschließlich der Prävention der damit verbundenen Folgeerkrankungen.
   Besondere Aspekte in diesem Zusammenhang sind
   - Hyperglykämia,
   - Verbesserung der Insulinresistenz,
   - Verbesserung der Glucosetoleranz,
   - Schutz der ß-Zellen der Bauchspeicheldrüse
   - Prävention makro- und mikrovaskulärer Erkrankungen
2. Übergewicht und dessen Folgen wie beispielsweise Dyslipidemien, Atherosklerose, koronare Herzkrankheit, zerebrovaskuläre Erkrankungen usw., insbesondere (jedoch nicht darauf beschränkt) die, die durch einen oder mehrere der folgenden Faktoren gekennzeichnet sind:
   - hohe Plasmatriglyceridkonzentrationen, hohe postprandiale Plasmatriglyceridkonzentrationen,
   - niedrige HDL-Cholesterinkonzentration
   - niedrige ApoA-Lipoproteinkonzentrationen
   - hohe LDL-Cholesterinkonzentrationen
   - kleine dichte LDL-Cholesterinpartikel hohe ApoB-Lipoproteinkonzentrationen
3. Verschiedene andere Leiden, die mit dem metabolischen Syndrom bzw. Syndrom X assoziert sein können, wie
   - Zunahme des Bauchumfangs
   - Dyslipidämie (z.B. Hypertriglyceridämie und/oder niedriges HDL)
   - Insulinresistenz
   - Hyperkoagulabilität
   - Hyperurikämie
   - Mikroalbuminämie
   - Thrombosen, hyperkoagulabile und prothrombotische Zustände (arteriell und venös)
   - Bluthochdruck
   - Herzinsuffizienz, beispielsweise (jedoch nicht darauf beschränkt), nach Herzinfarkt, hypertensiver Herzkrankheit oder Kardiomyopathie
4. Primäre Hypertriglyceridämie oder sekundäre Hypertriglyceridämien nach familiärer Retikulohistiozytose
   Lipoproteinlipasemangel
   Hyperlipoproteinämien
   Apolipoproteinmangel (z.B. ApoCII- oder ApoE-Mangel)
5. Genetisch bedingte verringerte Aktivität von Glucokinase, insbesondere dem so genannten MODY2
6. Krankheiten bzw. Leiden, die mit neurologischen, psychiatrischen oder Immunerkrankungen bzw. -leiden in Zusammenhang stehen

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 100 mg, typischerweise von 1 ng bis 100 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im Allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im Allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:
Alle Antidiabetika, die in der Roten Liste 2006, Kapitel 12 genannt sind; alle Abmagerungsmittel/Appetitzügler, die in der Roten Liste 2006, Kapitel 1 genannt sind; alle Lipidsenker, die in der Roten Liste 2006, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus® (siehe www.lantus.com) oder HMR 1964 oder Levemir® (insulin detemir) oder solche, wie sie in W02005005477 (Novo Nordisk) beschrieben sind, schnell wirkende Insuline (siehe US 6,221,633), inhalierbare Insuline, wie z. B. Exubera ® oder orale Insuline, wie z. B. IN-105 (Nobex) oder Oral-Iyn™ (Generex Biotechnology), GLP-1-Derivate wie z.B. Exenatide, Liraglutide oder diejenigen die in WO 98/08871, WO2005027978, W02006037811, W02006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die oral wirksamen hypoglykämischen Wirkstoffe umfassen vorzugsweise Sulfonylharnstoffe,
Biguanidine,
Meglitinide,
Oxadiazolidindione,
Thiazolidindione,
Glukosidase-Inhibitoren,
Hemmstoffe der Glykogenphosphorylase,
Glukagon-Antagonisten,
Glukokinaseaktivatoren,
Inhibitoren der Fructose-1,6-bisphosphatase
Modulatoren des Glukosetransporters-4 (GLUT4),
Inhibitoren der Glutamin-Fructose-6-Phosphat-Amidotransferase (GFAT),
GLP-1-Agonisten, Kaliumkanalöffner, wie z.B. diejenigen, die in WO 97/26265 und WO 99/03861 von Novo Nordisk A/S offenbart wurden oder die, die in WO2006045799 beschrieben sind (Solvay),
Inhibitoren der Dipeptidylpeptidase-IV (DPP-IV),
Insulin-Sensitizer, Inhibitoren von Leberenzymen, die an der Stimulation der Glukoneogenese und/oder
Glykogenolyse beteiligt sind,
Modulatoren der Glukoseaufnahme, des Glukosetransports und der Glukoserückresorption,
Hemmstoffe der 11ß-HSD1,
Inhibitoren der Protein-Tyrosin-Phosphatase-1B (PTP 1B),
Modulatoren des natrium-abhängigen Glukosetransporters 1 oder 2 (SGLT1, SGLT2), den Fettstoffwechsel verändernde Verbindungen wie antihyperlipidämische Wirkstoffe und
antilipidämische Wirkstoffe,
Verbindungen, die die Nahrungsmitteleinnahme verringern,
Verbindungen, die die Thermogenese erhöhen,
PPAR- und RXR-Modulatoren (Retinoid X Rezeptor) und
Wirkstoffe, die auf den ATP-abhängigen Kaliumkanal der Betazellen wirken.

Bei einer Ausführungsform der Erfindung wird die Verbindungen der Formel I in Kombination mit einem TR-β Agonisten (Thyroid Rezeptor) verabreicht.

Bei einer Ausführungsform der Erfindung wird die Verbindungen der Formel I in Kombination mit einem HMGCoA-Reduktase Inhibitor (Hydroxymethylglutaryl-coenzyme A) wie Simvastatin, Fluvastatin, Pravastatin, Lovastatin, Atorvastatin, Cerivastatin, Rosuvastatin, L-659699 verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, W02005042692, WO2005005453), MD-0727 (Microbia Inc., W02005021497, W02005021495) oder mit Verbindungen, wie in WO2002066464 (Kotobuki Pharmaceutical Co. Ltd.) oder W02005044256 oder WO2005062824 (Merck & Co.) oder W02005061451 und WO2005061452 (AstraZeneca AB) und WO2006017257 (Phenomix) oder W02005033100 (Lipideon Biotechnology AG) beschrieben, verabreicht.

Bei einer Ausführungsform der Erfindung wird die Verbindung der Formel I in Kombination mit Vytorin^{™}, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Fenofibrat verabreicht.

Bei einer weiteren Ausführungsform der Erfindung wird die Verbindung der Formel I in Kombination mit einer festen Kombination von Fenofibrat mit Rosuvastatin verabreicht.

Bei einer Ausführungsform der Erfindung wird die Verbindung der Formel I in Kombination mit ISIS-301012, einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem PPAR gamma Agonisten (Peroxisome proliferator-activated receptors), wie z.B. Rosiglitazon, Pioglitazon, JTT-501, Gl 262570, R-483, CS-011 (Rivoglitazon) verabreicht.

Bei einer Ausführungsform der Erfindung wird die Verbindung der Formel I in Kombination mit Competact^{™}, einer festen Kombination von Pioglitazon Hydrochlorid mit Metformin Hydrochlorid, verabreicht.

Bei einer Ausführungsform der Erfindung wird die Verbindung der Formel I in Kombination mit duetactTM, einer festen Kombination von Pioglitazon Hydrochlorid mit Glimepirid, verabreicht.

Bei einer Ausführungsform der Erfindung wird die Verbindung der Formel I in Kombination mit Avandamet®, einer festen Kombination von Rosiglitazon Maleat mit Metformin Hydrochlorid, verabreicht.

Bei einer Ausführungsform der Erfindung wird die Verbindung der Formel I in Kombination mit PPAR alpha Agonisten, wie z.B. GW9578, GW-590735, K-111, LY-674, KRP-101, DRF-10945 verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, oder wie in PCT/US 00/11833, PCT/US 00/11490, DE10142734.4 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht

Bei einer weiteren Ausführungsform der Erfindung wird die Verbindung der Formel I in Kombination mit einem Aktivator der AMP-aktivierten Proteinkinase (AMPK), wie z. B. A-769662 oder solchen Verbindungen wie sie in US20050038068 beschrieben sind, verabreicht.

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit einem MTP-Inhibitor (Microsomal Triglyzerid-Transfer-Protein), wie z.B. Implitapide , BMS-201038, R-103757 oder solchen wie in WO2005085226, WO2005121091, WO2006010423 beschrieben, verabreicht.

Bei einer Ausführungsform der Erfindung wird die Verbindung der Formel I in Kombination mit einem 5HT Agonisten (Serotonin reuptake) verabreicht.

Bei einer Ausführungsform der Erfindung wird die Verbindung der Formel I in Kombination mit einem CETP-Inhibitor (Cholesterinestertransferprotein), wie z.B. Torcetrapib oder JTT-705 oder solchen wie sie in WO2006002342, WO2006010422, WO2006012093 beschrieben sind, verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin, Colesevelam, verabreicht.

Bei einer Ausführungsform der Erfindung wird die Verbindung der Formel I in Kombination mit einem LDL-Rezeptorinducer (Low-Density-Lipoprotein - siehe US 6,342,512), wie z.B. HMR1171, HMR1586, oder solchen wie in WO2005097738 beschrieben, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor (Acyl-CoA:cholesterol acyltransferase), wie z.B. Avasimibe oder SMP-797, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, ß-Caroten oder Selen verabreicht.

Bei einer Ausführungsform der Erfindung wird die Verbindung der Formel I in Kombination mit einem Vitamin, wie z. B. Vitamin B6 oder Vitamin B12 verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor (Adenosintriphosphat-Citrat-Lyase), wie z.B. SB-204990, verabreicht.

Bei einer Ausführungsform der Erfindung wird die Verbindung der Formel I in Kombination mit einem TNF agonists (Tumor necrosis factor) verabreicht.

Bei einer Ausführungsform der Erfindung wird die Verbindung der Formel I in Kombination mit einem Squalen Synthetase Inhibitor, wie z.B. BMS-188494 oder wie in WO2005077907 beschrieben, verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Aqonisten des GPR109A (HM74A Rezeptor Agonisten), wie z.B. Nicotinsäure oder "extended release niacin" in Verbindung mit MK-0524A oder solchen Verbindungen, wie sie in WO2006045565, WO2006045564, WO2006069242 beschrieben sind, verabreicht.

Bei einer anderen Ausführungsform der Erfindung wird die Verbindung der Formel I in Kombination mit einem Aqonisten des GPR116, wie sie z.B. in WO2006067531, WO2006067532 beschrieben sind, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform wird die Verbindung der Formel I in Kombination mit einem Sulfonylharnstoff, wie z.B. Tolbutamid, Glibenclamid, Glipizid oder Glimepirid verabreicht. Bei einer Ausführungsform wird die Verbindung der Formel I in Kombination mit einer die Insulinsekretion verstärkende Substanz, wie z. B. KCP-265 (WO2003097064), verabreicht.

Bei einer Ausführungsform wird die Verbindung der Formel I in Kombination mit Agonisten des glucose-abhängigen insulinotropischen Rezeptors (GDIR) wie z. B. APD-668 verabreicht

Bei einer Ausführungsform wird die Verbindung der Formel I in Kombination mit einem Biguanid, wie z.B. Metformin, verabreicht.

Bei wieder einer anderen Ausführungsform wird die Verbindung der Formel I in Kombination mit einem Meglitinid, wie z.B. Repaglinide oder Nateglinid, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform wird die Verbindung der Formel I in Kombination mit einem α-Glukosidase-Inhibitor, wie z.B. Miglitol oder Acarbose, verabreicht.

Bei einer Ausführungsform wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932 beschrieben, verabreicht.

Bei einer Ausführungsform wird die Verbindung der Formel I in Kombination mit Glukagon-Rezeptor-Antagonisten, wie z.B. A-770077 oder NNC-25-2504 oder wie in WO2004100875, WO2005065680, beschrieben, verabreicht.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031, WO2004072066, WO2005080360, WO2005044801, WO2006016194, WO2006058923 beschrieben sind, verabreicht.

Bei einer Ausführungsform wird die Verbindung der Formel I in Kombination mit einem Inhibitor der Glukoneogenese, wie z. B. FR-225654, verabreicht.

Bei einer Ausführungsform wird die Verbindung der Formel I in Kombination mit Inhibitoren der Fructose-1,6-bisphosphatase (FBPase) wie z.B. CS-917 (MB-06322) oder MB-07803 oder solchen wie sie in WO2006023515 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X, KRP-104, DP-893 oder wie sie in WO2003074500, WO2003106456, WO200450658, WO2005058901, WO2005012312, WO2005/012308, WO2006039325, WO2006058064, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform wird die Verbindung der Formel I in Kombination mit Januvia^{™}, einer festen Kombination von Sitagliptin Phosphat mit Metformin hydrochlorid, verabreicht.

Bei einer Ausführungsform wird die Verbindung der Formel I in Kombination mit Hemmstoffen der 11-beta-Hydroxysteroid-Dehydrogenase-1 (11β-HSD1), wie z. B. BVT-2733, JNJ-25918646, INCB-13739 oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO200401141 WO2004033427, WO2004041264, WO2004037251, WO2004056744, WO2004058730, WO2004065351, WO2004089367, WO2004089380, WO2004089470-71, WO2004089896, WO2005016877, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109 beschrieben sind, verabreicht.

Bei einer Ausführungsform wird die Verbindung der Formel I in Kombination mit Inhibitoren der Protein-Tyrosin-Phosphatase-1B (PTP1B), wie sie z. B. in WO200119830-31, WO200117516, WO2004506446, WO2005012295, WO2005116003, PCT/EP2005/005311, PCT/EP2005/005321, PCT/EP2005/007151, PCT/EP2005/01294 oder DE 10 2004 060542.4 beschrieben sind, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268 und SAR 7226 oder wie sie z. B. in WO2004007517, WO200452903, WO200452902, PCT/EP2005/005959, WO2005085237, JP2004359630, WO2005121161, WO200601850, WO2006035796, WO2006062224, WO2006058597 oder von A. L. Handlon in Expert Opin. Ther. Patents (2005) 15(11), 1531-1540 beschrieben sind, verabreicht.

Bei einer Ausführungsform wird die Verbindung der Formel I in Kombination mit Modulatoren des GPR40 verabreicht.

Bei einer Ausführungsform wird die Verbindung der Formel I in Kombination mit Modulatoren des GPR119b, wie sie z. B. in WO2004041274 beschrieben sind, verabreicht.

Bei einer Ausführungsform wird die Verbindung der Formel I in Kombination mit Modulatoren des GPR119, wie sie z. B. in WO2005061489 (PSN-632408) beschrieben sind, verabreicht. Bei einer Ausführungsform wird die Verbindung der Formel I in Kombination mit Inhibitoren der hormon-sensitiven Lipase (HSL), wie z. B. in WO2005073199 beschrieben, verabreicht.

Bei einer Ausführungsform wird die Verbindung der Formel I in Kombination mit Hemmstoffen der Acetyl-CoA Carboxylase (ACC) wie z. B. solchen wie in WO199946262, WO200372197, WO02003072197, WO2005044814, WO2005108370, JP2006131559 beschrieben, verabreicht.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, PCT/EP2005/005346, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117 beschrieben.

Bei einer Ausführungsform wird die Verbindung der Formel I in Kombination mit einem Inhibitor der Protein Kinase C beta (PKC beta), wie z. B. Ruboxistaurin, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022553, WO2005097129 beschrieben sind, verabreicht.

Bei einer Ausführungsform wird die Verbindung der Formel I in Kombination mit Modulatoren des Glucocorticoidrezeptors, wie sie z. B. in WO2005090336 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten (Neuropeptid Y) wie z.B. Naphthalin-1-sulfonsäure-{4-[(4-amino-quinazolin-2-ylamino)-methyl]-cyclohexylmethyl}-amid Hydrochlorid (CGP 71683A); NPY-5 Rezeptorantagonisten wie L-152804, S-2367 oder wie sie z. B. in WO2006001318 beschrieben sind;
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424 beschrieben sind;
CB1R (Cannabinoid Rezeptor 1) Antagonisten (wie z.B. Rimonabant, SR147778, SLV-319, AVE-1625, MK-0364 oder Salze davon oder solche Verbindungen wie sie in z. B. EP 0656354, WO 00/15609, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006047516, WO2006060461, WO2006067428, WO2006067443, WO2006087480, WO2006087476, WO2006100208, WO2006106054, WO2006111849, WO2006113704, WO2007009705, WO2007017124, WO2007017126, WO2007018459, WO2007016460, WO2007020502, WO2007026215, WO2007028849, WO2007031720, WO2007031721, WO2007036945, WO2007038045, WO2007039740, US20070015810, WO2007046548, WO2007047737 beschrieben sind);
Cannabinoid Rezeptor 1 / Cannabinoid Rezeptor 2 (CB1/CB2) modulierende Verbindungen wie sie z.B. in WO2007001939, WO2007044215, WO2007047737 beschrieben sind;
MC4-Agonisten (Melanocortin-4 receptor-Agonisten z.B. 1-Amino-1,2,3,4-tetrahydronaphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, EP1538159, WO2004072076, WO2004072077, WO2006021655-57 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224 beschrieben sind);
Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893 beschrieben sind);
CRF-Antagonisten (Corticotropin-Releasing Factor -Antagonisten, z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
Agonisten des beta-3 Adrenoceptors wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553 beschrieben sind;
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, WO2004012648, WO2003015769, WO2004072025, WO2005070898, WO2005070925, WO2004039780, WO2003033476, WO2002006245, WO2002089729, WO2002002744, WO2003004027, FR2868780, WO2006010446, WO2006038680, WO2006044293, WO2006044174 beschrieben sind);
CCK-A Agonisten (Cyclic pseudopeptide cholecystokinin-A Agonisten, wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180) oder solchen, wie sie in WO2005116034 beschrieben sind;
Serotonin-Wiederaufnahme-Inhibitoren (z.B. Dexfenfluramine);
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. Lorcaserin Hydrochlorid (APD-356) oder BVT-933 oder solche, wie sie in WO200077010, WO20077001-02, WO2005019180, WO2003064423, WO200242304, WO2005082859 beschrieben sind);
5-HT6 Rezeptor Antagonisten, wie sie z.B. in WO2005058858 beschrieben sind;
Bombesin-Rezeptor Agonisten (BRS-3 Agonisten;
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695));
Growth Hormone Secretagogue Receptor Antagonisten (Ghrelin Antagonisten) wie z. B. A-778193 oder solchen, wie sie in WO2005030734 beschrieben sind;
TRH-Agonisten (Thyrotrophin-releasing hormone - siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Dopamine Agonist, z.B. Bromocriptin, Doprexin);
Lipase/Amylase-Inhibitoren (z.B. WO 00/40569); Inhibitoren der Diacylglycerol O-Acyltransferasen (DGATs) wie z. B. BAY-74-4113 oder wie z. B. in US2004/0224997, WO2004094618, WO200058491, WO2005044250, WO2005072740, JP2005206492, WO2005013907, WO2006004200, WO2006019020, WO2006064189 beschrieben;
Inhibitoren der Fettsäuresynthase (FAS) wie z.B. C75 oder solchen, wie in WO2004005277 beschrieben;
Oxyntomodulin;
Oleoyl-Estron
oder Agonisten des Schilddrüsenhorrnonrezeptors (thyroid hormone receptor agonists) wie z. B: KB-2115 oder solche, wie in WO20058279, WO200172692, WO200194293, WO2003084915, WO2004018421, WO2005092316 beschrieben, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIRT1, eines Mitglieds der humanen Sirtuinenzymfamilie.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer Ausführungsform ist der weitere Wirkstoff ein Diphenylazetidinonderivat, wie z.B. in US 6,992,067 oder US 7,205,290 beschrieben.

Bei einer Ausführungsform wird die Verbindung der Formel I in Kombination mit Ballaststoffen, vorzugsweise unlöslichen Ballaststoffen (siehe z.B. Carob/ Caromax® (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax® kann in einer Zubereitung erfolgen, oder durch getrennte Gabe von Verbindungen der Formel I und Caromax®. Caromax® kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

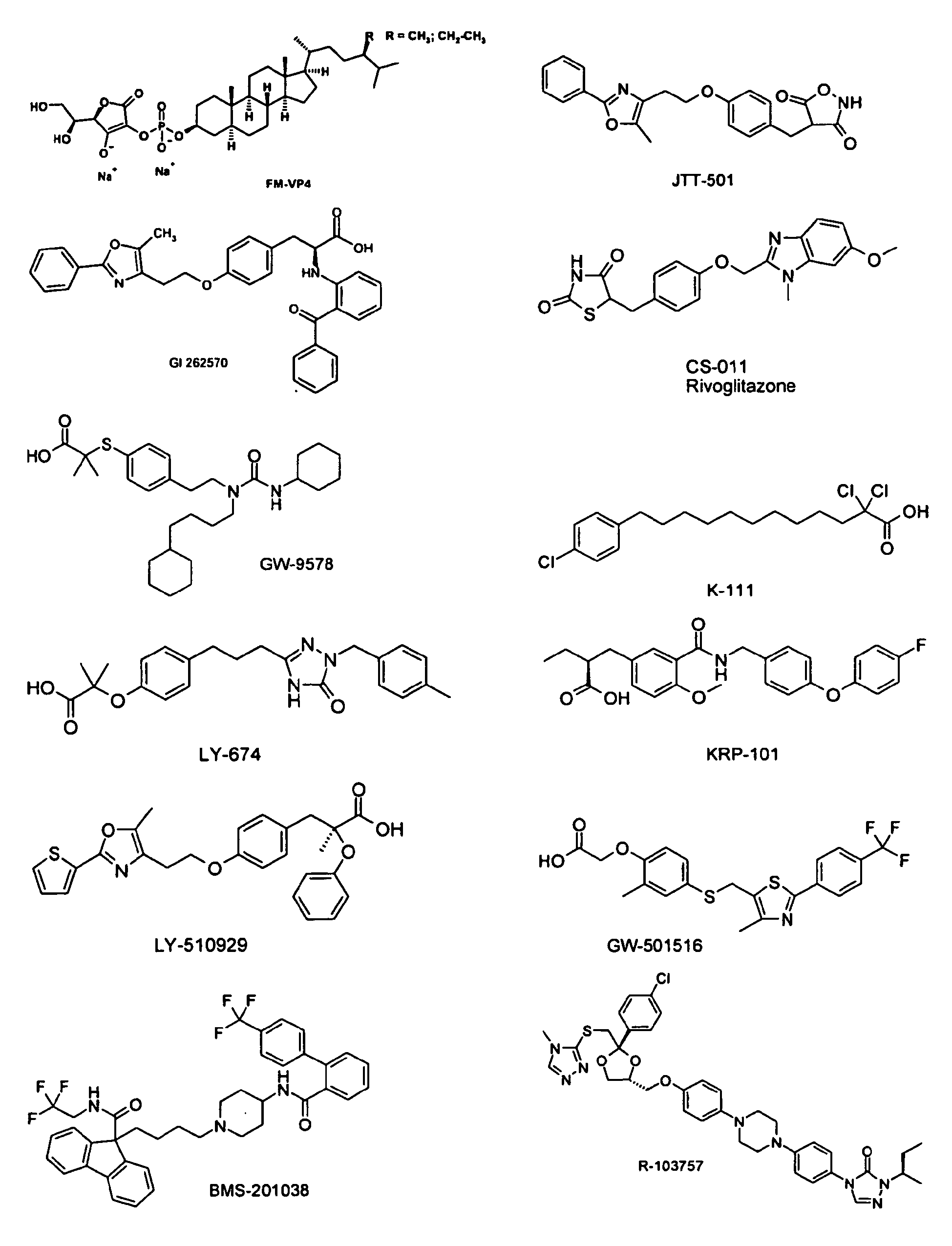

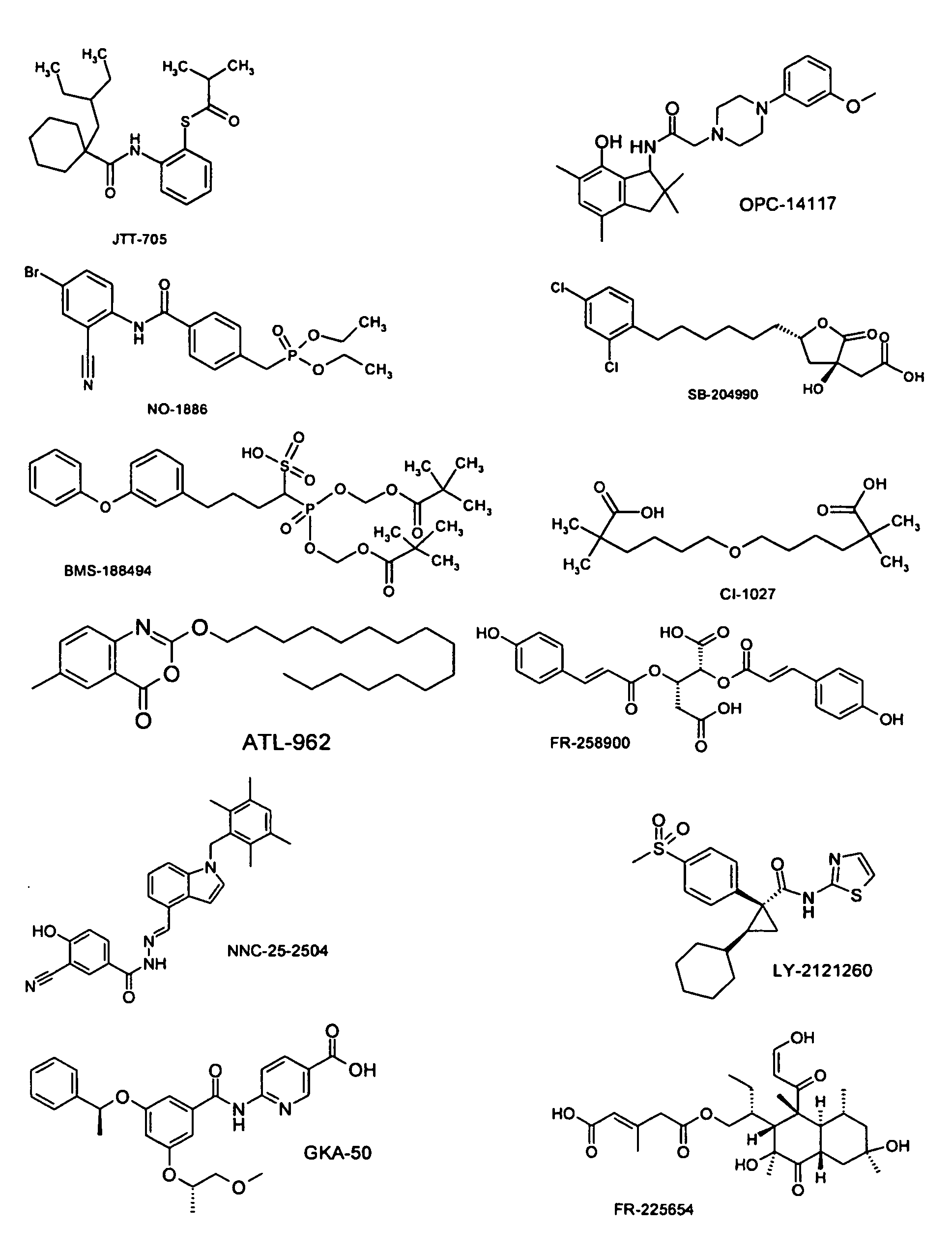

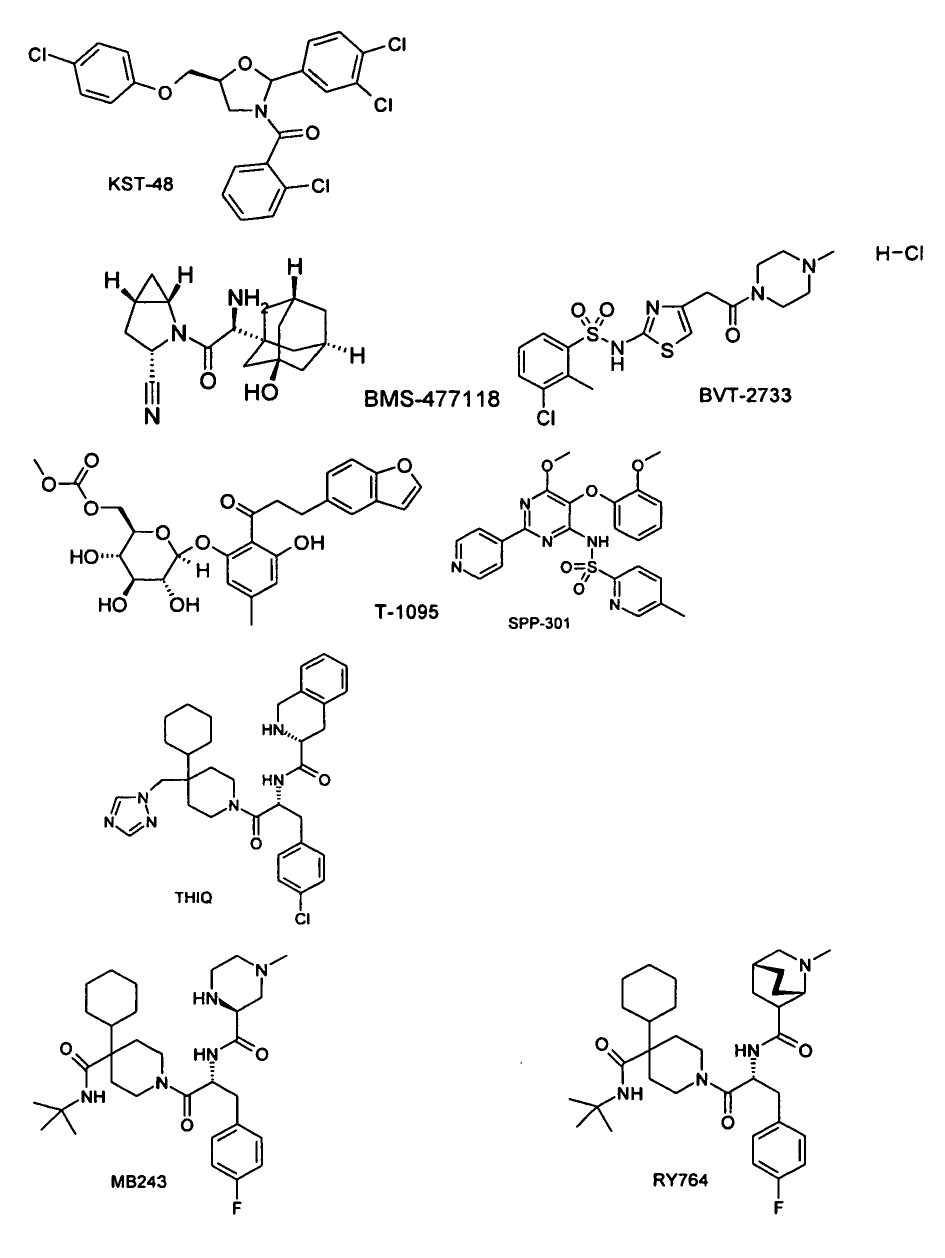

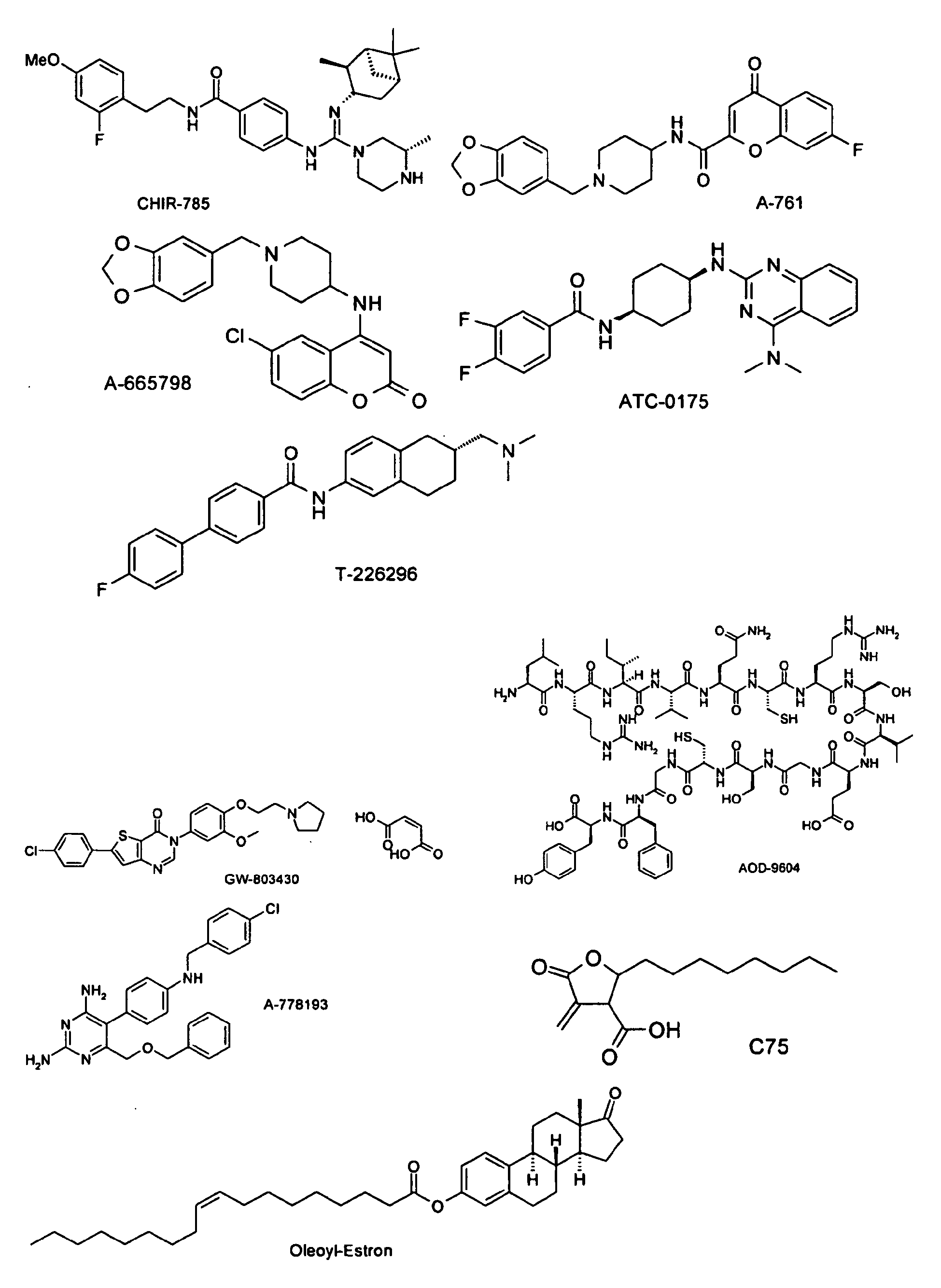

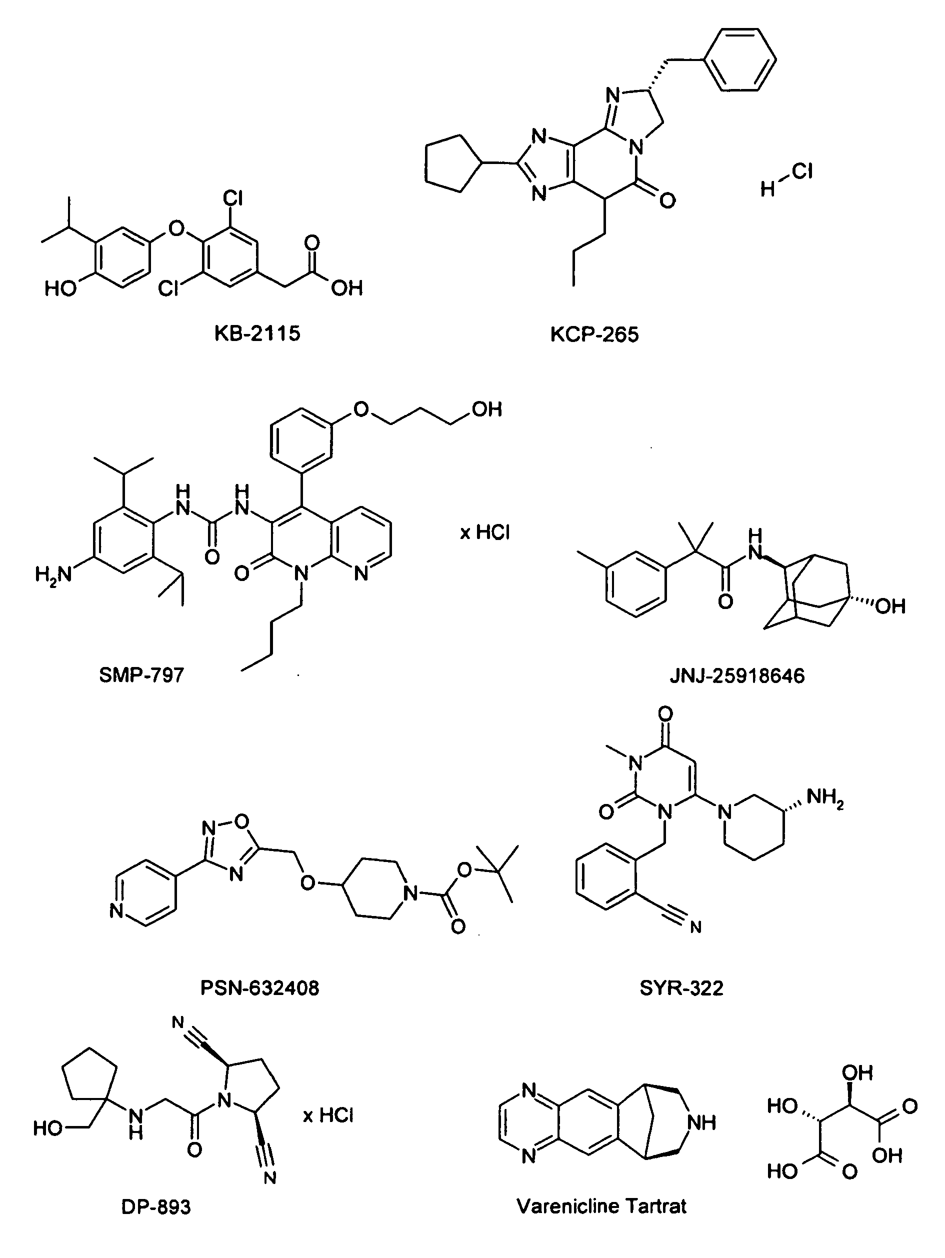

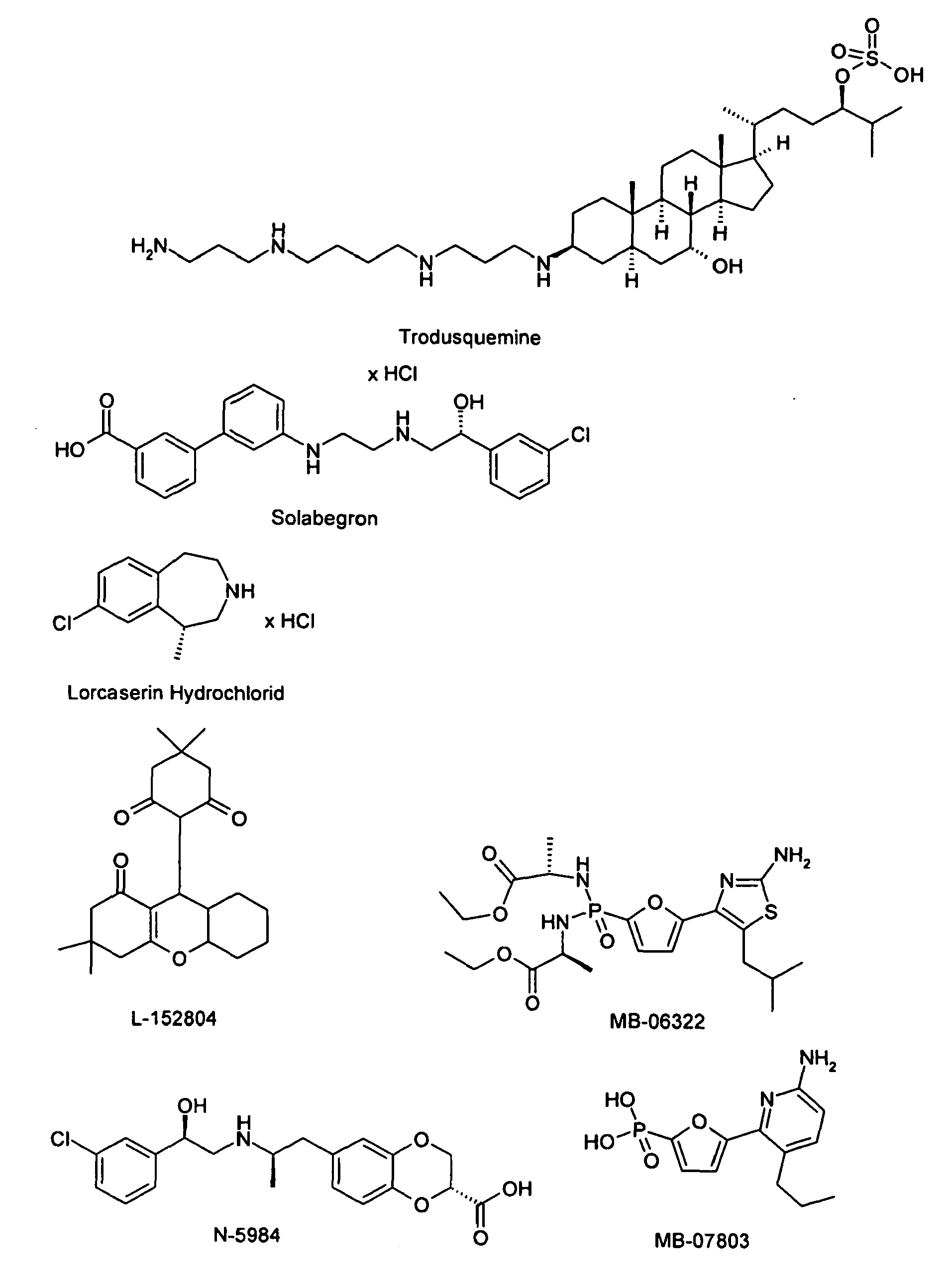

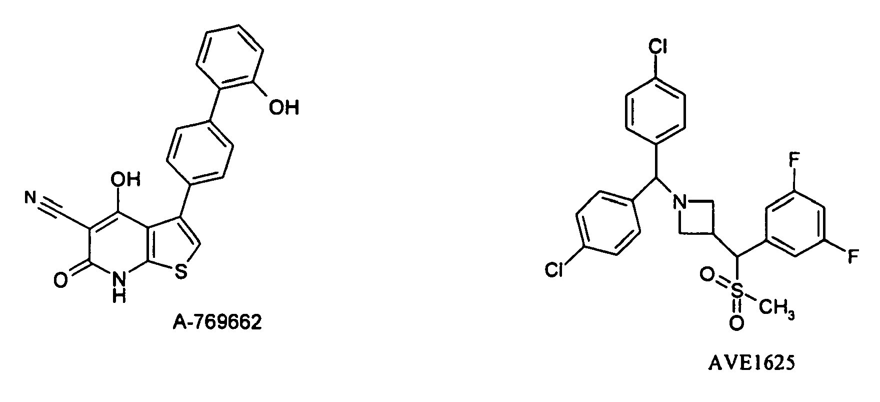

Die nachfolgend aufgeführten Beispiele dienen zur Erläuterung der Erfindung, ohne diese jedoch einzuschränken. Tabelle 1:

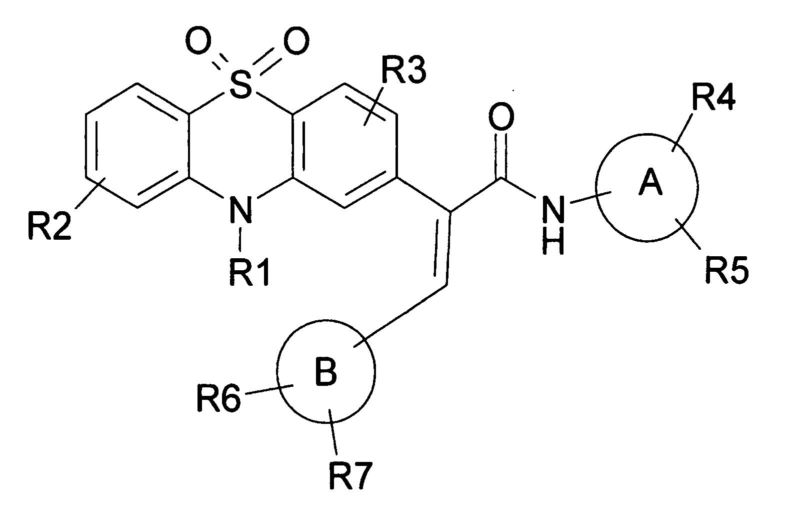

| **Beispiel** | **R1** | **R2** | **R3** | **A** | **R4** | **R5** | **B** | **R6** | **R7** |
|---|---|---|---|---|---|---|---|---|---|
| **1** | CH₃ | H | H | 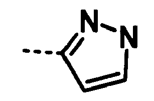 | 1-CH₃ | H | 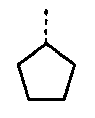 | H | H |
| **2** | CH₃ | H | H | 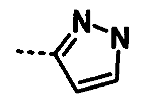 | 1-CH₂Ph | H | 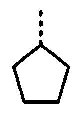 | H | H |
| **3** | H | H | H | 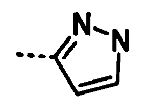 | 1-CH₃ | H | 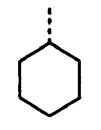 | 4-'=O' | H |
| **4** | H | H | H | 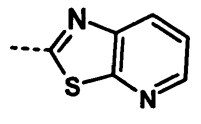 | 5-OCH₃ | H | 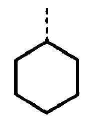 | 4-'=O' | H |
| **5** | H | H | H | 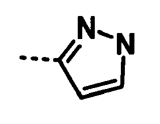 | 1-CH₃ | H | 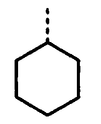 | 4-'=O' | H |

Die gestrichelte Linie in den Resten A und B zeigt den Anknüpfungspunkt der Bindung am Ring.

Die Wirksamkeit der Verbindungen wurde wie folgt getestet:
Enzymatischer Test von Glucokinase-Aktivatoren
Humane Glucokinase
Humane Glucokinase wird als Fusionsprotein mit Glutathion-S-Transferase (GST) in E. coli B121 exprimiert und durch eine Affinitätschromatographie aufgereinigt. Durch Verdauung mit Faktor Xa wird GSH abgespalten und das Glucokinase-Polypeptid beginnend mit Ser-6 erhalten. Letzteres wird chromatographisch aufgereinigt. Eine typische Präparation der Glucokinase besitzt bei Raumtemperatur eine spezifische Aktivität von 30 U/mg Protein.

### Enyzymatischer Test

Die Aktivität von Glucokinase und der Einfluss von Verbindungen auf diese Aktivität werden durch einen gekoppelten optischen Test bei 25 °C bestimmt. Das Testvolumen beträgt 100 µl. Die Testzusammensetzung ist: 25 mM HEPES/NaOH (Merck; #110110) pH 7, 25 mM KCl (Merck; #04933), 2 mM MgCl₂ (Merck; #05833), 1 mM Dithiothreitol (Merck; #112013), 1 mM NAD (Sigma; #N1511), 5 mM Glucose (Merck; #108337), 1 mM ATP (Sigma; #A2383), 0,1 % (w/v) Rinderserumalbumin (Merck; #112018), 0,002 U Glucokinase-Präparation und 3,2 U Glucose-6-Phosphat Dehydrogenase (Sigma; #G8529). Ferner enthält der Ansatz eine Testverbindung. Die Testverbindungen sind jeweils in 10 mM DMSO gelöst und werden bei Endkonzentrationen von 0 µM, 0,1 µM, 0,3 µM, 1 µM, 3 µM, 10 µM, 30 µM und 100 µM getestet. Die Endkonzentration an DMSO im Test beträgt 1 % (v/v). Die Reaktion wird durch die Zugabe von ATP gestartet. Die Absorption des Ansatzes bei 340 nm wird unmittelbar nach der Zugabe von ATP und dann 25 min später mit einem Multiwellplattenphotometer (Firma Labsystems, Multiskan Ascent) bestimmt. Die Änderung der Absorption in diesem Zeitraum wird berechnet.

### Auswertung:

Die Rohdaten der Extinktionsänderungen werden in ein Microsoft Excel-File transferiert. Der Wert für 0 µM Testverbindung wird als 100 % gesetzt. Dosis-Wirkungskurven werden mit dem Programm XL.Fit nach Vorgabe des Herstellers (Firma IDBS) berechnet. Als EC150 wird die Konzentration einer Testverbindung definiert, die eine Steigerung der enzymatischen Aktivität um 50 % hervorruft. Die maximal fache Stimulation entspricht dem Verhältnis der größten Extinktionsänderung im Konzentrationsbereich einer Testverbindung zu der Änderung der Absorption ohne Testsubstanz.

**Tabelle 2: Biologische Aktivität**

| **Beispiel Nr** | **EC₁₅₀ [**µ**M]** | **Fold induction** |
|---|---|---|
| **1** | **1.70** | **3.2** |
| **4** | **0.2** | **4.8** |

Aus den Messdaten der Tabelle ist abzulesen, dass die erfindungsgemäßen Verbindungen eine Aktivierung von Glukokinase bewirken. Diese Verbindungen eignen sich damit insbesondere zur Senkung des Blutzuckerspiegel und zur Behandlung von Diabetes.

### Verfahren

Die erfindungsgemäßen Verbindungen der Formel I können entsprechend dem folgenden Reaktionsschema hergestellt werden:

### Verfahren A:

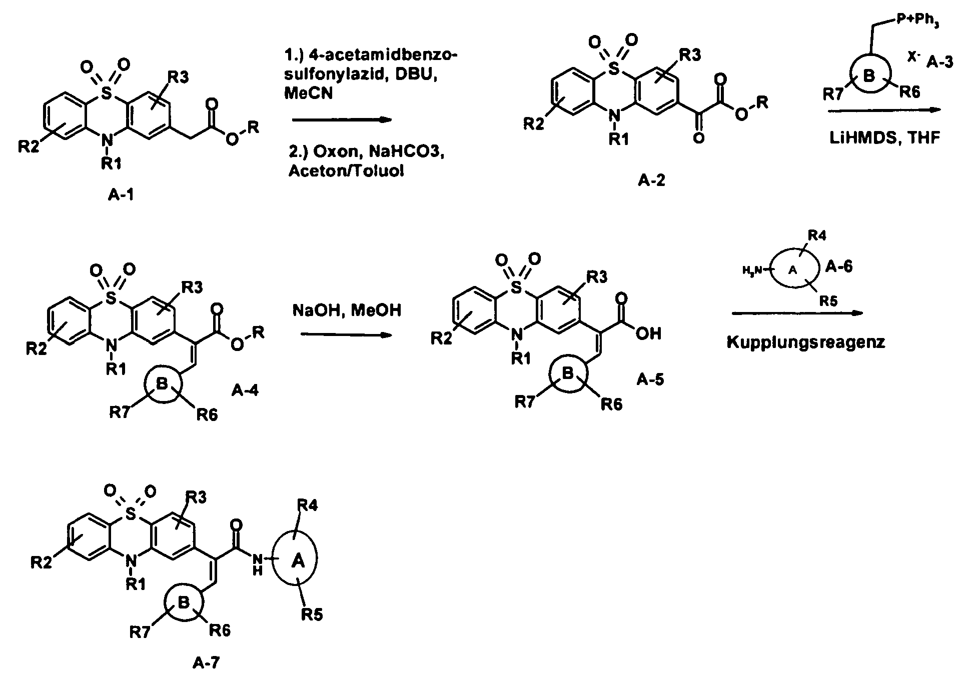

Ein Phenothiazinester (R = Methyl oder Ethyl) der allgemeinen Formel A-1 (Herstellung beschrieben in DE2007-002), wobei R1, R2 und R3 die oben genannten Bedeutungen haben, wird in einem polaren aprotischen Lösungsmittel wie zum Beispiel Acetonitril mit einer Base wie zum Beispiel 1,8-Diazabicyclo[5.4.0]undee-7-ene bei Raumtemperatur deprotoniert und anschließend bei tiefer Temperatur (-20 °C - 0°C) mit 4- Acetaminobenzolsulfonylazid versetzt und anschließend bei Raumtemperatur reagieren lassen. Die dabei entstehende Diazoverbindung wird nicht isoliert sondern gleich durch Zugabe eines Oxidationsmittel wie zum Beispiel Oxon in einem Lösungsmittelgemisch wie zum Beispiel Aceton/Toluol in Anwesenheit einer Base, wie zum Beispiel Natriumhydrogencarbonat, zur Ketoverbindung der allgemeinen Formel A-2 umgesetzt. Die Ketoverbindung der allgemeinen Formel A-2 wird mit einem Wittigreagenz, welches durch Freisetzung des entsprechenden Wittigsalzes der allgemeinen Formel A-3 durch eine Base, wie zum Beispiel Lithiumhexamethyldisilazid in einem polar aprotischen Lösungsmittel wie Tetrahydrofuran, zum α,β-ungesättigtem Ester der allgemeinen Formel A-4 umgesetzt. Die estergruppe der Verbindung der allgemeinen Formel A-4 wird mittels einer Base wie zum Beispiel Natronlauge in einem polar protischen Lösungsmittelgemisch wie Methanol/Wasser zur Carbonsäure der allgemeinen Formel A-5 hydrolysiert. Unter Einwirkung eines Kupplungsreagenzes, wie zum Beispiel O-[Cyan(ethoxycarbonyl)methylenamino]-1,1,3,3-tetramethyluronium-tetrafluoroborat (TOTU) oder [Dimethylamino-([1,2,3]triazolo[4,5-b]pyridin-3-yloxy)-methylene]-dimethyl-ammonium hexafluoro phosphate (HATU) / [1,2,3]Triazolo[4,5-b]pyridin-3-ol (HOAT) in Gegenwart einer Base, wie zum Beispiel Diisopropylethylamin in einem polar aprotischen Lösungsmittel wie N,N-Diemethylformamid wird die Carbonsäure der allgemeinen Formel A-5 mit dem Amin der allgemeinen Formel A-6, worin A, R4 und R5 die oben beschriebenen Bedeutungen haben, zum Amid der allgemeinen Formel A-7 umgesetzt. Die racemischen Verbindungen der allgemeinen Formel A-7 können durch Chromatographie an chiraler Phase in die Enantiomere getrennt werden.

Die Beispiele 1-5 wurden nach Verfahren A hergestellt.

Die verwendeten Abkürzungen stehen für:

| | |
|---|---|
| Ac | Acetyl |
| Bn | Benzyl |
| BOC | Tert-Butyloxycarbonyl |
| iBu | Isobutyl |
| tBu | tert-Butyl |
| BuLi | n-Butyllithium |
| DC | Dünnschichtchromatographie |
| DCI | direkte chemische Ionisation (bei MS) |
| DCM | Dichlormethan |
| DMAP | 4-N,N-Dimethylaminopyridin |
| DMF | N,N-Dimethylformamid |
| DMSO | Dimethylsulfoxid |
| EE | Ethylacetat |
| ent | Enantiomer / enantiomerenrein |
| EI | Elektronenstoß-Ionisation (bei MS) |
| eq | Äquivalent |
| ESI | Elektronenspray-Ionisation (bei MS) |
| Et | Ethyl |
| GC | Gaschromatographie |
| HATU | [Dimethylamino-([1,2,3]triazolo[4,5-b]pyridin-3-yloxy)-methylene]-dimethyl-ammonium hexafluoro phosphate |
| HOAT | [1,2,3]Triazolo[4,5-b]pyridin-3-ol |
| HPLC | Hochdruck-, Hochleistungsflüssigchromatographie |
| LiHMDS | Lithiumhexamethyldisilazid |
| LC-MS | Flüssigchromatographie-gekoppelte Massenspektroskopie |
| m | meta |
| M | molar |
| mCPBA | meta-Chlorperbenzoesäure |
| Me | Methyl |
| MeCN | Acetonitril |
| MS | Massenspektroskopie |
| NMR | Kernresonanzspektroskopie |
| o | ortho |
| Oxon | Kaliummonopersulfat-sulfat |
| p | para |
| Pd/C | Palladium auf Kohle |
| Ph | Phenyl |
| iPr | Isopropyl |
| nPr | n-Propyl |
| rac | racemisch / racemisches Gemisch |
| Rf | Retentionszeit (bei DC) |
| RP | Reversed Phase |
| tert | tertiär |
| THF | Tetrahydrofuran |
| TOTU | O-[Cyan(ethoxycarbonyl)methylenamino]-1,1,3,3-tetramethyluronium-tetrafluoroborat |

### Beispielsynthesen nach Verfahren A

### Beispiel 1

### (E)-3-Cyclopentyl-2-(10-methyl-5,5-dioxo-5,10-dihydro-phenothiazin-2-yl)-N-(1-methyl-1H-pyrazol-3-yl)-acrylamid

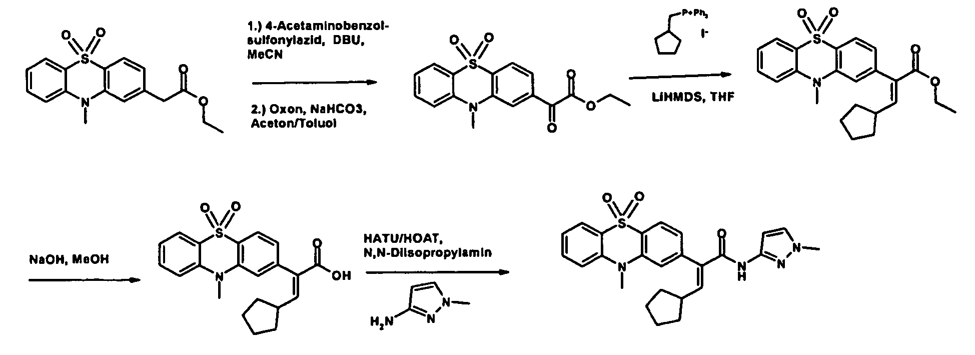

### (10-Methyl-5,5-dioxo-5,10-dihydro-phenothiazin-2-yl)-oxo-essigsäureethylester

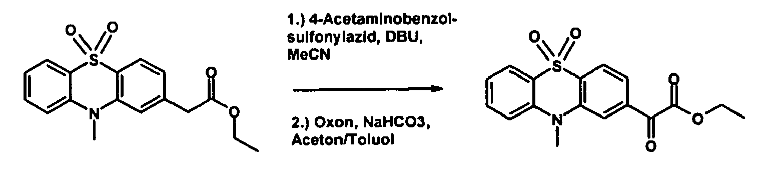

2.24 g (10-Methyl-5,5-dioxo-5,10-dihydro-phenothiazin-2-yl)-essigsäureethylester wird in 50 ml Acetonitril gelöst und mit 0.71 ml DBU versetzt. Nach 15 minütigem Rühren bei Raumtemperatur werden unter Eiskühlung 1.95 g 4-Acetaminobenzolsulfonylazid zugegeben.

Das Reaktionsgemisch rührt zwölf Stunden bei Raumtemperatur. (Rf der Diazoverbindung in n-Heptan:Ethylacetat = 2:1 Rf= 0.15). Es werden 50 ml Toluol, 35 ml Aceton und 50 ml Wasser zur Reaktionsmischung zugegeben gefolgt von 40.3 g Oxon und 21.78 g NaHCO₃. Man rührt eine Stunde bei Raumtemperatur nach. Das Reaktionsgemisch wird durch Zugabe von 200 ml Wasser verdünnt und dreimal mit je 300 ml Ethylacetat extrahiert. Die vereinigten organischen Phasen werden über MgSO₄ getrocknet und anschließend im Vakuum eingeengt. Der resultierende Rückstand wird an Kieselgel mit dem Elunes 100 % n-Heptan => 100% Ethylacetat als linearer Gradient gereinigt. Man erhält 1.87 g (10-Methyl-5,5-dioxo-5,10-dihydro-phenothiazin-2-yl)-oxo-essigsäureethylester.

C17H15NO5S (345.38), LCMS(ESI): 346.1 (M+H⁺), Rf(n-Heptan:Ethylacetat = 2:1) = 0.11.

### (E)-3-Cyclopentyl-2-(10-methyl-5,5-dioxo-5, 1 0-dihydro-phenothiazin-2-yl)-acrylsäureethylester

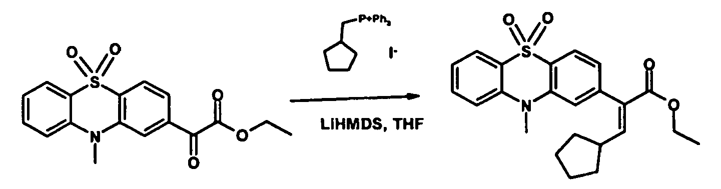

1.47 ml 1,1,1,3,3,3-Hexamethyldisilazan werden in 20 ml Tetrahydrofuran unter Argon gelöst. Unter Eiskühlung werden 2.57 ml n-Buthyllithium (2.5 M in n-Hexan) zugetropft und 30 Minuten bei 0°C nachgerührt.

Anschließend wird diese Lösung zu einer gerührten Suspension 2.53 g Cyclopentylmethyltriphenyl-phosphoniumiodid in 60 ml Tetrahydrofuran unter Eiskühlung zugetropft. Das Reaktionsgemisch wird 45 Minuten bei 0°C gerührt, dann werden 1.85 g (10-Methyl-5,5-dioxo-5,10-dihydro-phenothiazin-2-yl)-oxo-essigsäureethylester gelöst in 20 ml THF zugetropft und eine Stunde bei 0°C gerührt. Das Kühlbad wird entfernt und man lässt langsam auf Raumtemperatur erwärmen. Der Reaktionsansatz wird über Nacht bei Raumtemperatur gerührt. Dann werden 30 ml gesättigte Kochsalzlösung zugegeben und das Gemisch dreimal mit je 100 ml Ethylacetat extrahiert. Die vereinigten organischen Phasen werden über MgSO₄ getrocknet und anschließend im Vakuum eingeengt. Der Rückstand wird an Kieselgel mit dem Eluens n-Heptan:Ethylacetat (100%:0%)=> n-Heptan:Ethylacetat (40%:60%) gereinigt. Man erhält 1.58 g (E)-3-Cyclopentyl-2-(10-methyl-5,5-dioxo-5,10-dihydro-phenothiazin-2-yl)-acrylsäureethylester als farblosen Feststoff.

C23H25NO4S (411.52), LCMS(ESI): 412.2 (M+H⁺), Rf(n-Heptan:Ethylacetat = 2:1) = 0.27.

### (E)-3-Cyclopentyl-2-(10-methyl-5,5-dioxo-5,10-dihydro-phenothiazin-2-yl)-acrylsäure

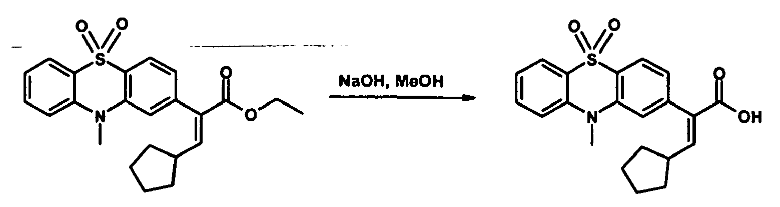

1.58 g (E)-3-Cyclopentyl-2-(10-methyl-5,5-dioxo-5,10-dihydro-phenothiazin-2-yl)-acrylsäureethylester werden in 130 ml Methanol gelöst und mit 13.44 ml 2 M NaOH Lösung versetzt. Das Reaktionsgemisch wird zwei Stunden unter Rückfluss zum Sieden erhitzt. Das Methanol wird im Vakuum entfernt und das Reaktionsgemisch durch Zugabe von 2N Salzsäure auf pH 4 eingestellt. Der ausgefallene Feststoff wird abgesaugt und im Hochvakuum getrocknet. Man erhält 1.46 g (E)-3-Cyclopentyl-2-(10-methyl-5,5-dioxo-5,10-dihydro-phenothiazin-2-yl)-acrylsäure.

C21H21NO4S (383.47), LCMS(ESI): 384.1 (M+H⁺).

### (E)-3-Cyclopentyl-2-(10-methyl-5,5-dioxo-5,10-dihydro-phenothiazin-2-yl)-N-(1-methyl-1H-pyrazol-3-yl)-acrylamid

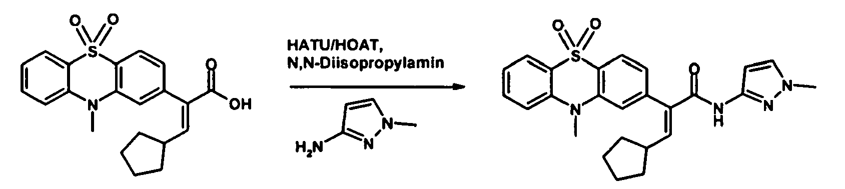

480 mg (E)-3-Cyclopentyl-2-(10-methyl-5,5-dioxo-5,10-dihydro-phenothiazin-2-yl)-acrylsäure, 122 mg kommerziell erhältliches 1-Methyl-1H-pyrazol-3-ylamin und 0.76 ml N,N-Diisopropylethylamin werden in 7 ml Dimethylformamid gelöst. Man gibt 571 mg HATU und 204 mg HOAT hinzu und rührt eine Stunde bei Raumtemperatur. Danach wird das Reaktionsgemisch durch Zugabe von 100 ml Ethylacetat verdünnt und fünfmal mit je 30 ml Wasser gewaschen. Die organische Phase wird über MgSO₄ getrocknet und anschließend das Lösungsmittel im Vakuum entfernt. Der Rückstand wird an Kieselgel mit dem Eluens n-Heptan (100%)=> :Ethylacetat (100%) gereinigt.. Man erhält 360 mg (E)-3-Cyclopentyl-2-(10-methyl-5,5-dioxo-5,10-dihydro-phenothiazin-2-yl)-N-(1-methyl-1H-pyrazol-3-yl)-acrylamid.

C25H26N4O3S (462.57), LCMS(ESI): 463.2(M+H⁺), Rf(Ethylacetat) = 0.37.

### Beispiel 2

### (E)-N-(1-Benzyl-1H-pyrazol-3-yl)-3-cyclopentyl-2-(10-methyl-5,5-dioxo-5,10-dihydro-phenothiazin-2-yl)-acrylamid

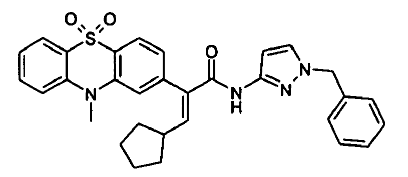

Analog zu Beispiel 1 erhält man aus (E)-3-Cyclopentyl-2-(10-methyl-5,5-dioxo-5,10-dihydro-phenothiazin-2-yl)-acrylsäure und kommerziell erhältlichem 1-Benzyl-1H-pyrazol-3-ylamin (E)-N-(1-Benzyl-1H-pyrazol-3-yl)-3-cyclopentyl-2-(10-methyl-5,5-dioxo-5,10-dihydro-phenothiazin-2-yl)-acrylamid.

C31H30N4O3S (538.67), LCMS(ESI): 539.3(M+H⁺), Rf(Ethylacetat) = 0.69.

### Beispiel 3

### (E)-2-(5,5-Dioxo-5,10-dihydro-phenothiazin-2-yl)-N-(1-methyl-1H-pyrazol-3-yl)-3-(4-oxo-cyclohexyl)-acrylamid

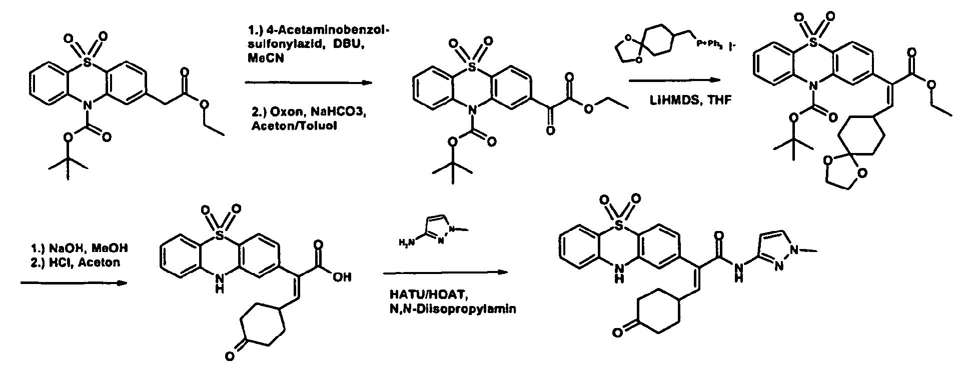

### 2-Ethoxyoxalyl-5,5-dioxo-5H-phenothiazine-10-carbonsäure-tert-butylester

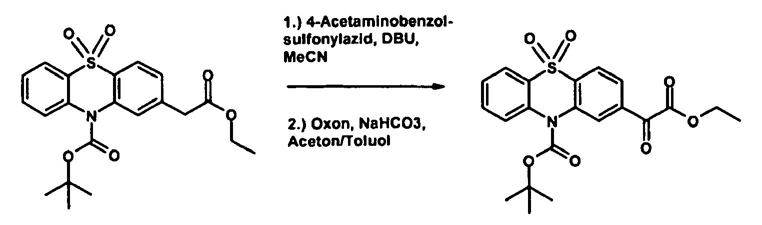

2.0 g 2-Ethoxycarbonylmethyl-5,5-dioxo-SH-phenothiazine-10-carbonsäure-tert-butylester werden in 35 ml Acetonitril gelöst und mit 0.51 ml DBU versetzt. Nach 15 minütigem Rühren bei Raumtemperatur werden unter Eiskühlung 1.38 g 4-Acetaminobenzolsulfonylazid zugegeben. Das Reaktionsgemisch rührt zwölf

Stunden bei Raumtemperatur. (Rf der Diazoverbindung in n-Heptan:Ethylacetat = 2:1 Rf= 0.36). Es werden 35 ml Toluol, 25 ml Aceton und 35 ml Wasser zur Reaktionsmischung zugegeben gefolgt von 28.57 g Oxon und 15.44 g NaHCO₃. Man rührt eine Stunde bei Raumtemperatur nach. Das Reaktionsgemisch wird durch Zugabe von 300 ml Wasser verdünnt und dreimal mit je 200 ml Ethylacetat extrahiert. Die vereinigten organischen Phasen werden über MgSO₄ getrocknet und anschließend im Vakuum eingeengt. Der resultierende Rückstand wird an Kieselgel mit dem Elunes 90 % n-Heptan /10% Ethylacetat => 100% Ethylacetat als linearer Gradient gereinigt. Man erhält 1.84 g 2-Ethoxyoxalyl-5,5-dioxo-5H-phenothiazine-10-carbonsäure-tert-butylester.

C21H21N07S (431.47), LCMS(ESI): 332.1 (M-BOC+H⁺), Rf(n-Heptan:Ethylacetat = 2:1) = 0.29.

### (1,4-Dioxa-spiro[4.5]dec-8-ylmethyl)-triphenyl-phosphoniumiodid

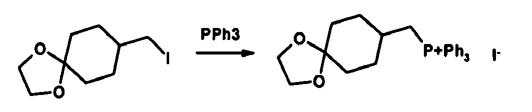

5.54 g 8-Iodomethyl-1,4-dioxa-spiro[4.5]decan und 5.15 g Triphenylphosphin werden in 32 ml Acetonitril gelöst und 12 Stunden unter Rückfluss zum Sieden erhitzt. Man rührt zwei Tage bei Raumtemperatur nach, dann wird das im Vakuum auf etwa 15 ml Lösungsvolumen eingeengt und 30 ml Diethylether versetzt. Unter kräftigem Rühren in einem Eisbad beginnnt sich ein Niederschlag abzuscheiden. Der Niederschlag wird abgesaugt und im Vakuum getrocknet. Man erhält 3.86 g (1,4-Dioxa-spiro[4.5]dec-8-ylmethyl)-triphenyl-phosphoniumiodid.

C21H21N07S (544.42), LCMS(ESI): 417.2 (M⁺).

### 2-[(E)-2-(1,4-Dioxa-spiro[4.5]dec-8-yl)-1-ethoxycarbonyl-vinyl]-5,5-dioxo-5H-phenothiazin-10-carbonsäure-tert-butylester

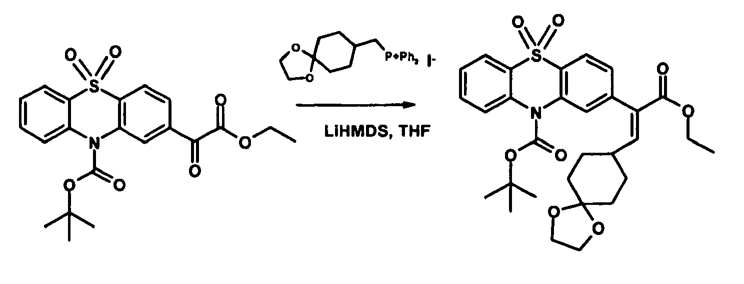

0.76 ml 1,1,1,3,3,3-Hexamethyldisilazan werden in 20 ml Tetrahydrofuran unter Argon gelöst. Unter Eiskühlung werden 1.32 ml n-Buthyllithium (2.5 M in n-Hexan) zugetropft und 30 Minuten bei 0°C nachgerührt. Anschließend wird diese Lösung zu einer gerührten Suspension 1.19 g (1,4-Dioxa-spiro[4.5]dec-8-ylmethyl)-triphenyl-phosphoniumiodid in 80 ml Tetrahydrofuran unter Eiskühlung zugetropft. Das Reaktionsgemisch wird 45 Minuten bei 0°C gerührt, dann werden 1.80g 2-Ethoxyoxalyl-5,5-dioxo-5H-phenothiazin-10-carbonsäure-tert-butylester gelöst in 20 ml THF zugetropft und eine Stunde bei 0°C gerührt. Das Kühlbad wird entfernt und man lässt langsam auf Raumtemperatur erwärmen. Der Reaktionsansatz wird über Nacht bei Raumtemperatur gerührt. Dann werden 30 ml gesättigte Kochsalzlösung zugegeben und das Gemisch dreimal mit je 100 ml Ethylacetat extrahiert. Die vereinigten organischen Phasen werden über MgSO₄ getrocknet und anschließend im Vakuum eingeengt. Der Rückstand wird an Kieselgel mit dem Eluens 100 %n-Heptan => Ethylacetat 100% gereinigt. Man erhält 1.14 g 2-[(E)-2-(1,4-Dioxa-spiro[4.5]dec-8-yl)-1-ethoxycarbonyl-vinyl]-5,5-dioxo-5H-phenothiazin-10-carbonsäure-tert-butylester als hellgelben Feststoff.

C30H35NO8S (569.68), LCMS(ESI): 587.2 (M+NH4⁺), 514.2 (M-tert-Butyl+H⁺),470.2 (M-BOC+H⁺), Rf(n-Heptan:Ethylacetat = 2:1) = 0.16.

### (E)-2-(5,5-Dioxo-5,10-dihydro-phenothiazin-2-yl)-3-(4-oxo-cyclohexyl)-acrylsäure

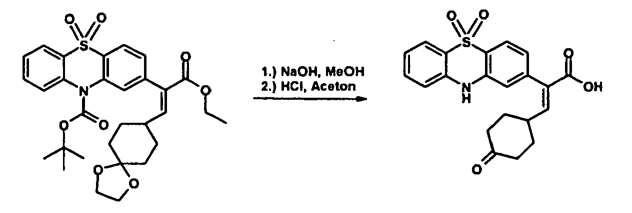

1.14 g 2-[(E)-2-(1,4-Dioxa-spiro[4.5]dec-8-yl)-1-ethoxycarbonyl-vinyl]-5,5-dioxo-5H-phenothiazin-10-carbonsäure-tert-butylester werden in 130 ml Methanol gelöst und mit 7.0 ml 2 M NaOH Lösung versetzt. Das Reaktionsgemisch wird zwei Stunden unter Rückfluss zum Sieden erhitzt. Das Methanol wird im Vakuum entfernt und das Reaktionsgemisch durch Zugabe von konzentrierter Salzsäure auf pH 4 eingestellt. Der ausgefallene Feststoff wird in 200 ml Ethylacetat gelöst, über MgSO₄ getrocknet und anschließend das Lösungsmittel im Vakuum entfernt. Der resultierende Rückstand wird in 20 ml Aceton gelöst und mit 4 ml 50%iger Salzsäurelösung versetzt. Das Reaktionsgemisch wird 30 Minuten bei Raumtemperatur gerührt. Das Aceton wird im Vakuum entfernt und der Rückstand in 100 ml Wasser und 100 ml Ethylacetat aufgenommen. Die organische Phase wird über MgSO₄ getrocknet und anschließend das Lösungsmittel im Vakuum entfernt. Man erhält 840 mg (E)-2-(5,5-Dioxo-5,10-dihydro-phenothiazin-2-yl)-3-(4-oxo-cyclohexyl)-acrylsäure.

C21H19NO5S (397.45), LCMS(ESI): 398.2 (M+H⁺).

### (E)-2-(5,5-Dioxo-5,10-dihydro-phenothiazin-2-yl)-N-(1-methyl-1H-pyrazol-3-yl)-3-(4-oxo-cyclohexyl)-acrylamid

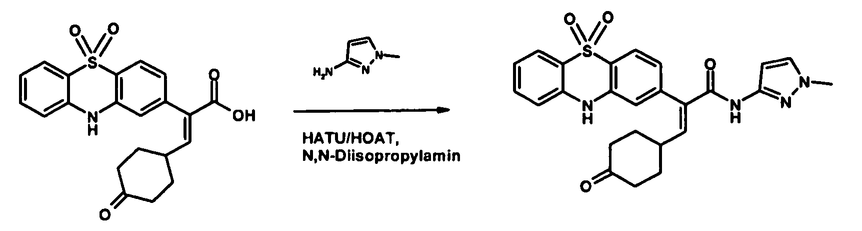

276 mg (E)-2-(5,5-Dioxo-5,10-dihydro-phenothiazin-2-yl)-3-(4-oxo-cyclohexyl)-acrylsäure, 101 mg kommerziell erhältliches 1-Methyl-1H-pyrazol-3-ylamin und 0.61 ml N,N-Diisopropylethylamin werden in 10 ml Dimethylformamid gelöst. Man gibt 318 mg HATU und 114 mg HOAT hinzu und rührt zwei Stunden bei Raumtemperatur. Danach wird das Reaktionsgemisch durch Zugabe von 100 ml Ethylacetat verdünnt und fünfmal mit je 30 ml Wasser gewaschen. Die organische Phase wird über MgSO₄ getrocknet und anschließend das Lösungsmittel im Vakuum entfernt. Der Rückstand wird an Kieselgel mit dem Eluens n-Heptan (100%)=> :Ethylacetat (100%) gereinigt.. Man erhält 134 mg (E)-2-(5,5-Dioxo-5,10-dihydro-phenothiazin-2-yl)-N-(1-methyl-1H-pyrazol-3-yl)-3-(4-oxo-cyclohexyl)-acrylamid. C25H24N4O4S (476.56), LCMS(ESI): 477.3 (M+H⁺), 518.2 (M+MeCN+H⁺), Rf(Ethylacetat) = 0.10.

### Beispiel 4

### (E)-2-(5,5-Dioxo-5,10-dihydro-phenothiazin-2-yl)-N-(5-methoxy-thiazolo[5,4-b]pyridin-2-yl)-3-(4-oxo-cyclohexyl)-acrylamid

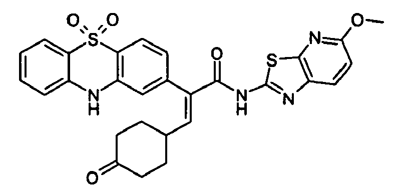

Analog zu Beispiel 3 erhält man aus (E)-2-(5,5-Dioxo-5,10-dihydro-phenothiazin-2-yl)-3-(4-oxo-cyclohexyl)-acrylsäure und 5-Methoxy-thiazolo[5,4-b]pyridin-2-ylamin (E)-2-(5,5-Dioxo-5,10-dihydro-phenothiazin-2-yl)-N-(5-methoxy-thiazolo[5,4-b]pyridin-2-yl)-3-(4-oxo-cyclohexyl)-acrylamid.

C28H24N4O5S2 (560.66), LCMS(ESI): 561.1(M+H⁺), Rf(Ethylacetat) = 0.40.

### Beispiel 5

### (E)-N-(1-Benzyl-1H-pyrazol-3-yl)-2-(5,5-dioxo-5,10-dihydro-phenothiazin-2-yl)-3-(4-oxo-cyclohexyl)-acrylamid

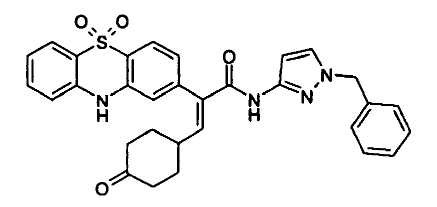

Analog zu Beispiel 3 erhält man aus (E)-2-(5,5-Dioxo-5,10-dihydro-phenothiazin-2-yl)-3-(4-oxo-cyclohexyl)-acrylsäure und 1-Benzyl-1H-pyrazol-3-ylamin (E)-N-(1-Benzyl-1H-pyrazol-3-yl)-2-(5,5-dioxo-5,10-dihydro-phenothiazin-2-yl)-3-(4-oxo-cyclohexyl)-acrylamid. C31H28N4O4S (552.66), LCMS(ESI): 553.4(M+H⁺), Rf(Ethylacetat) = 0.39.

## Patentansprüche

1. Verbindungen der Formel I, 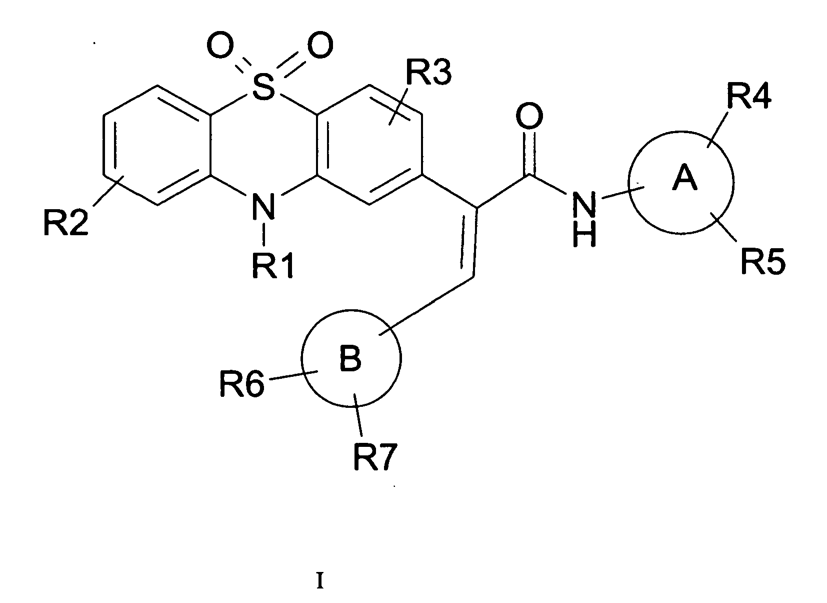 worin bedeuten
R1 H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl;
R2, R3 unabhängig voneinander H, F, Cl, Br, CN, NO₂, (C₀-C₆)-Alkylen-COO-(C₀- C₆)-Alkyl, (C₀-C₆)-Alkylen-O-(C₀-C₆)-Alkyl, (C₁-C₆)-Alkyl, (C₀-C₆)-Alkylen- CO-(C₁-C₆)-Alkyl, (C₀-C₆)-Alkylen-Phenyl, SCF₃, SF₅, SCH₃;
R4, R5 unabhängig voneinander H, F, Cl, Br, CN, SCN, NO₂, (C₀-C₆)-Alkylen-COO- (C₀-C₆)-Alkyl, -CO-COO-(C₀-C₆)-Alkyl, (C₀-C₆)-Alkylen-O-(C₀-C₆)-Alkyl, (C₁-C₆)-Alkyl, (C₀-C₆)-Alkylen-CO-(C₁-C₆)-Alkyl, (C₀-C₆)-Alkylen- CONH(C₀-C₆)-Alkyl, (C₀-C₆)-Alkylen-CON[(C₀-C₆)-Alkyl]₂, (C₀-C₆)-Alkylen- NH(C₀-C₆)-Alkyl, (C₀-C₆)-Alkylen-NH-COO-(C₀-C₆)-Alkyl, (C₀-C₆)-Alkylen- CON[(C₀-C₆)-Alkyl]-O-(C₀-C₆)-Alkyl, (C₀-C₆)-Alkylen-N[(C₀-C₆)-Alkyl]₂, (C₀-C₆)-Alkylen-Aryl, SF₅, (C₀-C₆)-Alkyl-S(O)ₓ(C₁-C₆)-Alkyl, S(O)ₓ(C₁-C₆)- Alkylen-COO-(C₀-C₆)-Alkyl, S(O)ₓ(C₂-C₆)-Alkylen-O-(C₀-C₆)-Alkyl, -SO₂-NH-(C₀-C₆)-Alkyl, -SO₂-N-[(C₀- C₆)-Alkyl]₂, S(O)ₓ(C₀-C₆)-Alkylen-Heterocyclus, S(O)ₓ(C₁-C₆)-Alkylen-CO- Heterocyclus, -NH-SO₂-(C₁-C₆)-Alkyl, (C₀-C₆)-Alkylen-Cycloalkyl, (C₀-C₆)- Alkylen-Heterocyclus, (C₀-C₆)-Alkylen-Aryl;
R6, R7 unabhängig voneinander H, F, Cl, Br, CN, NO₂, =O, =S, =N-O-(C₀-C₆)-Alkyl, (C₀-C₆)-Alkylen-COO-(C₀-C₆)-Alkyl, (C₀-C₆)-Alkylen-O-(C₀-C₆)-Alkyl, (C₀- C₆)-Alkylen-O-CO-(C₁-C₆)-Alkyl, (C₁-C₆)-Alkyl, (C₀-C₆)-Alkylen-CO-(C₁- C₆)-Alkyl, (C₀-C₆)-Alkylen-Aryl, SF₅, S(O)ₓ-(C₁-C₆)-Alkyl;
A 5 bis 10 gliedriger Heterocyclus, wobei der Heterocyclus mit einem weiteren 5 bis 10 gliedrigen Ring anneliert sein kann;
B 4 bis 8 gliedriger Cycloalkylring, ein 4 bis 10 gliedriger Heterocyclus oder ein 6 bis 10 gliedriger Arylring;
wobei unter der Defintion -(C₀-C₆)-Alkyl jeweils verstanden wird, dass entweder ein Wasserstoff oder eine (C₁-C₆)-Alkyl Gruppe vorhanden sein kann,
sowie deren physiologisch verträgliche Salze.

2. Verbindungen der Formel I, gemäß Anspruch 1, **dadurch gekennzeichnet, dass** darin bedeuten
R1 H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl
R2, R3 unabhängig voneinander H, F, Cl, Br, CN, NO₂, (C₀-C₆)-Alkylen-COO-(C₀- C₆)-Alkyl, (C₀-C₆)-Alkylen-O-(C₀-C₆)-Alkyl, (C₁-C₆)-Alkyl, (C₀-C₆)-Alkylen- CO-(C₁-C₆)-Alkyl, (C₀-C₆)-Alkylen-Phenyl, SCF₃, SF₅, SCH₃;
R4, R5 unabhängig voneinander H, F, Cl, Br, CN, SCN, NO₂, (C₀-C₆)-Alkylen-COO- (C₀-C₆)-Alkyl, -CO-COO-(C₀-C₆)-Alkyl, (C₀-C₆)-Alkylen-O-(C₀-C₆)-Alkyl, (C₁-C₆)-Alkyl, (C₀-C₆)-Alkylen-CO-(C₁-C₆)-Alkyl, (C₀-C₆)-Alkylen- CONH(C₀-C₆)-Alkyl, (C₀-C₆)-Alkylen-CON[(C₀-C₆)-Alkyl]₂, (C₀-C₆)-Alkylen- NH(C₀-C₆)-Alkyl, (C₀-C₆)- Alkylen-NH-COO-(C₀-C₆)-Alkyl, (C₀-C₆)-Alkylen-CON[(C₀-C₆)-Alkyl]-O- (C₀-C₆)-Alkyl, (C₀-C₆)-Alkylen-N[(C₀-C₆)-Alkyl]₂, (C₀-C₆)-Alkylen-Aryl, SF₅, (C₀-C₆)-Alkyl-S(O)ₓ(C₁-C₆)-Alkyl, S(O)ₓ(C₁-C₆)-Alkylen-COO-(C₀-C₆)-Alkyl, S(O)ₓ(C₂-C₆)-Alkylen-O-(C₀-C₆)-Alkyl, -SO₂-NH-(C₀-C₆)-Alkyl, -SO₂-N-[(C₀- C₆)-Alkyl]₂, S(O)ₓ(C₀-C6)-Alkylen-Heterocyclus, S(O)ₓ(C₁-C₆)-Alkylen-CO- Heterocyclus, -NH-SO₂-(C₁-C₆)-Alkyl, (C₀-C₆)-Alkylen-Cycloalkyl, (C₀-C₆)- Alkylen-Heterocyclus, (C₀-C₆)-Alkylen-Aryl;
R6, R7 unabhängig voneinander H, F, Cl, Br, CN, NO₂, =O, =S, =N-O-(C₀-C₆)-Alkyl, (C₀-C₆)-Alkylen-COO-(C₀-C₆)-Alkyl, (C₀-C₆)-Alkylen-O-(C₀-C₆)-Alkyl, (C₀- C₆)-Alkylen-O-CO-(C₁-C₆)-Alkyl, (C₁-C₆)-Alkyl, (C₀-C₆)-Alkylen-CO-(C₁- C₆)-Alkyl, (C₀-C₆)-Alkylen-Aryl, SF₅, S(O)ₓ-(C₁-C₆)-Alkyl;
A 5 bis 10 gliedriger Heterocyclus, wobei der Heterocyclus mit einem weiteren 5 bis 10 gliedrigen Ring anneliert sein kann;
B 4 bis 8 gliedriger Cycloalkylring;
sowie deren physiologisch verträgliche Salze.

3. Verbindungen der Formel I, gemäß Anspruch 1 oder 2, **dadurch gekennzeichnet, dass** darin bedeuten
R1 H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl
R2, R3 H;
R4 (C₁-C₆)-Alkyl, O-(C₁-C₆)-Alkyl, (C₀-C₆)-Alkylen-Aryl;
R5 H;
R6, R7 unabhängig voneinander H, F, Cl, Br, CN, NO₂ =O, =S, =N-O-(C₀-C₆)-Alkyl, (C₀-C₆)-Alkylen-COO-(C₀-C₆)- Alkyl, (C₀-C₆)-Alkylen-O-(C₀-C₆)-Alkyl, (C₀-C₆)-Alkylen-O-CO-(C₁-C₆)- Alkyl, (C₁-C₆)-Alkyl, (C₀-C₆)-Alkylen-CO-(C₁-C₆)-Alkyl, (C₀-C₆)-Alkylen- Aryl, SF₅, S(O)ₓ-(C₁-C₆)-Alkyl;
A 5 bis 10 gliedriger Heterocyclus, wobei der Heterocyclus mit einem weiteren 5 bis 10 gliedrigen Ring anneliert sein kann;
B 4 bis 8 gliedriger Cycloalkylring;
sowie deren physiologisch verträgliche Salze.

4. Verbindungen gemäß einem oder mehreren der Ansprüche 1 bis 3, zur Anwendung als Arzneimittel.

5. Arzneimittel enthaltend eine oder mehrere der Verbindungen gemäß einem oder mehreren der Ansprüche 1 bis 3.

6. Arzneimittel enthaltend eine oder mehrere der Verbindungen gemäß einem oder mehreren der Ansprüche 1 bis 3 und mindestens einen weiteren Wirkstoff.

7. Arzneimittel, gemäß Anspruch 6, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, MTP-Inhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Modulatoren des GPR40, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten,, CB1-Rezeptor Antagonisten , MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, Diphenylazetidinonderivat, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

8. Verwendung der Verbindungen gemäß einem oder mehreren der Ansprüche 1 bis 3 zur Herstellung eines Medikamentes zur Behandlung des Diabetes.

9. Verwendung der Verbindungen gemäß einem oder mehreren der Ansprüche 1 bis 3 zur Herstellung eines Medikamentes zur Blutzuckersenkung.

10. Verwendung der Verbindungen gemäß einem oder mehreren der Ansprüche 1 bis 3 zur Herstellung eines Medikamentes zur Behandlung des metabolischen Syndroms.

11. Verwendung der Verbindungen gemäß einem oder mehreren der Ansprüche 1 bis 3 zur Herstellung eines Medikamentes zur Behandlung von Nikotinabhängigkeit.

12. Verwendung der Verbindungen gemäß einem oder mehreren der Ansprüche 1 bis 3 zur Herstellung eines Medikamentes zur Behandlung von Alkoholabhängigkeit.

13. Verwendung der Verbindungen gemäß einem oder mehreren der Ansprüche 1 bis 3 zur Herstellung eines Medikamentes zur Behandlung von ZNS-Erkrankungen.

14. Verwendung der Verbindungen gemäß einem oder mehreren der Ansprüche 1 bis 3 zur Herstellung eines Medikamentes zur Behandlung von Schizophrenie.

15. Verwendung der Verbindungen gemäß einem oder mehreren der Ansprüche 1 bis 3 zur Herstellung eines Medikamentes zur Behandlung von Alzheimer.

16. Verfahren zur Herstellung eines Arzneimittels enthaltend eine oder mehrere der Verbindungen gemäß einem oder mehreren der Ansprüche 1 bis 3, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 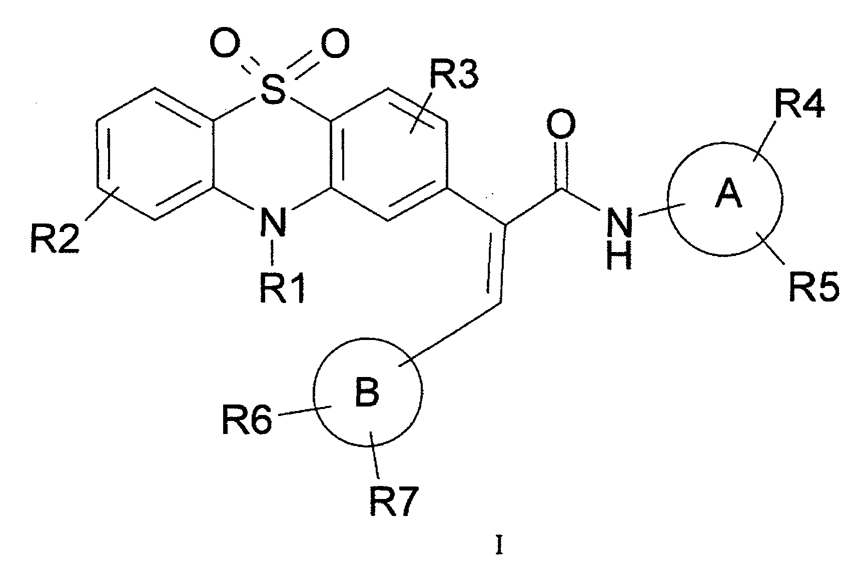 in which
R1 is H, (C₁-C₆)-alkyl, (C₀-C₆)-alkylene-aryl, CO- (C₁-C₆)-alkyl, (C₂-C₆)-alkylene-COO-(C₀-C₆)- alkyl , (C₂-C₆) -alkylene-O- (C₁-C₆) -alkyl ;
R2, R3 independently of one another are H, F, Cl, Br, CN, NO₂, (C₀-C₆) -alkylene-COO- (C₀-C₆) -alkyl, (C₀- C₆) -alkylene-O- (C₀-C₆) -alkyl, (C₁-C₆) -alkyl, (C₀- C₆) -alkylene-CO- (C₁-C₆) -alkyl, (C₀-C₆) -alkylene- phenyl, SCF₃, SF₅, SCH₃;
R4, R5 independently of one another are H, F, Cl, Br, CN, SCN, NO₂, (C₀-C₆)-alkylene-COO-(C₀-C₆)-alkyl, -CO-COO- (C₀-C₆) -alkyl, (C₀-C₆)-alkylene-O- (C₀- C₆-alkyl, (C₁-C₆)-alkyl, (C₀-C₆) -alkylene-CO- (C₁-C₆)-alkyl, (C₀-C₆) -alkylene-CONH (C₀-C₆)-alkyl, (C₀-C₆)-alkylene-CON[(C₀-C₆)-alkyl]₂, (C₀- C₆)-alkylene-NH (C₀-C₆)-alkyl, (C₀-C₆)-alkylene- NH-COO- (C₀-C₆) -alkyl, (C₀-C₆) -alkylene-CON [(C₀- C₆)-alkyl] -O- (C₀-C₆) -alkyl, (C₀-C₆) -alkylene- N [(C₀-C₆)-alkyl]₂ , (C₀-C₆) -alkylene-aryl, SF₅, (C₀-C₆)-alkyl-S(O)ₓ(C₁-C₆)-alkyl, S(O)ₓ(C₁-C₆)-alkylene-COO-(C₀-C₆)-alkyl, S(O)ₓ(C₂-C₆)- alkylene-O-(C₀-C₆)-alkyl, -SO₂-NH (C₀-C₆) -alkyl, -SO₂-N-[(C₀-C₆)-alkyl]₂ , S (O)ₓ(C₀-C₆)-alkylene- heterocycle, S(O)ₓ(C₁-C₆)-alkylene-CO- heterocycle, -NH-SO₂- (C₁-C₆)-alkyl, (C₀-C₆)- alkylene-cycloalkyl, (C₀-C₆)-alkylene- heterocycle, (C₀-C₆)-alkylene-aryl;
R6, R7 independently of one another are H, F, Cl, Br, CN, NO₂, =O, =S, =N-O-(C₀-C₆)-alkyl, (C₀-C₆)- alkylene-COO-(C₀-C₆)-alkyl, (C₀-C₆-alkylene-O- (C₀-C₆)-alkyl, (C₀-C₆) -alkylene-O-CO- (C₁-C₆)-alkyl, (C₁-C₆)-alkyl, (C₀-C₆) -alkylene-CO- (C₁- C₆)-alkyl, (C₀-C₆)-alkylene-aryl, SF₅, S(O)ₓ-(C₁- C₆)-alkyl;
A is a 5- to 10-membered heterocycle, where the heterocycle may be fused to a further 5- to 10- membered ring;
B is a 4- to 8-membered cycloalkyl ring, a 4- to 10-membered heterocycle or a 6- to 10-membered aryl ring;
the definition - (C₀-C₆) -alkyl in each case being understood as meaning that either a hydrogen or a (C₁-C₆)-alkyl group may be present,
or a physiologically acceptable salt thereof.

2. The compound of the formula I as claimed in claim 1, wherein
R1 is H, (C₁-C₆) -alkyl, (C₀-C₆)-alkylene-aryl, CO- (C₁-C₆)-alkyl, (C₂-C₆) -alkylene-COO- (C₀-C₆)- alkyl, (C₂-C₆) -alkylene-O- (C₁-C₆) -alkyl
R2, R3 independently of one another are H, F, Cl, Br, CN, NO₂, (C₀-C₆)-alkylene-COO-(C₀-C₆)-alkyl, (C₀- C₆)-alkylene-O-(C₀-C₆)-alkyl, (C₁-C₆) -alkyl, (C₀- C₆)-alkylene-CO-(C₁-C₆)-alkyl, (C₀-C₆)-alkylene- phenyl, SCF₃ , SF₅, SCH₃;
R4, R5 independently of one another are H, F, Cl, Br, CN, SCN, NO₂, (C₀-C₆) -alkylene-COO-(C₀-C₆)-alkyl, -CO-COO- (C₀-C₆) -alkyl, (C₀-C₆) -alkylene-O- (C₀- C₆)-alkyl, (C₁-C₆)-alkyl, (C₀-C₆) -alkylene-CO- (C₁-C₆)-alkyl, (C₀-C₆)-alkylene-CONH (C₀-C₆)- alkyl, (C₀-C₆)-alkylene-CON[(C₀-C₆)-alkyl]₂, (C₀- C₆)-alkylene-NH (C₀-C₆)-alkyl, (C₀-C₆)-alkylene- NH-COO- (C₀-C₆) -alkyl, (C₀-C₆) -alkylene-CON [(C₀- C₆)-alkyl]-O-(C₀-C₆)-alkyl, (C₀-C₆)-alkylene- N[(C₀-C₆)-alkyl]₂, (C₀-C₆) -alkylene-aryl, SF₅, (C₀-C₆)-alkyl-S(O)ₓ(C₁-C₆)-alkyl, S(O)ₓ(C₁-C₆)- alkylene-COO-(C₀-C₆)-alkyl, S(O)ₓ(C₂-C₆)- alkylene-O-(C₀-C₆)-alkyl, -SO₂-NH-(C₀-C₆)-alkyl, -SO₂-N-[(C₀-C₆)-alkyl]₂, S(O)ₓ(C₀-C₆)-alkylene- heterocycle, S(O)ₓ(C₁-C₆)-alkylene-CO- heterocycle, -NH-SO₂-(C₁-C₆)-alkyl, (C₀-C₆)- alkylene-cycloalkyl, (C₀-C₆)-alkylene- heterocycle, (C₀-C₆)-alkylene-aryl;
R6, R7 independently of one another are H, F, Cl, Br, CN, NO₂ =O =S, =N-O- (C₀-C₆) -alkyl, (C₀-C₆)- alkylene-COO- (C₀-C₆) -alkyl, (C₀-C₆)-alkylene-O- (C₀-C₆)-alkyl, (C₀-C₆)-alkylene-O-CO-(C₁-C₆)- alkyl, (C₁-C₆)-alkyl, (C₀-C₆)-alkylene-CO-(C₁- C₆)-alkyl, (C₀-C₆)-alkylene-aryl, SF₅, S(O)ₓ-(C₁- C₆)-alkyl ;
A is a 5- to 10-membered heterocycle, where the heterocycle may be fused to a further 5- to 10- membered ring;
B is a 4- to 8-membered cycloalkyl ring;
or a physiologically acceptable salt thereof.

3. The compound of the formula I as claimed in claim 1 or 2, wherein
R1 is H, (C₁-C₆)-alkyl, (C₀-C₆)-alkylene-aryl, CO- (C₁-C₆)-alkyl, (C₂-C₆)-alkylene-COO-(C₀-C₆)-alkyl, (C₂-C₆)-alkylene-O-(C₁-C₆)-alkyl
R2, R3 are H;
R4 is (C₁-C₆)-alkyl, O-(C₁-C₆)-alkyl, (C₀-C₆)- alkylene-aryl;
R5 is H;
R6, R7 independently of one another are H, F, Cl, Br, CN, NO₂ =O, =S, =N-O-(C₀-C₆)-alkyl, (C₀-C₆)- alkylene-COO- (C₀-C₆) -alkyl, (C₀-C₆) -alkylene-O- (C₀-C₆)-alkyl, (C₀-C₆)-alkylene-O-CO-(C₁-C₆)- alkyl, (C₁-C₆)-alkyl, (C₀-C₆)-alkylene-CO-(C₁- C₆)-alkyl, (C₀-C₆)-alkylene-aryl, SF₅, S(O)ₓ-(C₁- C₆)-alkyl ;
A is a 5- to 10-membered heterocycle, where the heterocycle may be fused to a further 5- to 10- membered ring;
B is a 4- to 8-membered cycloalkyl ring;
or a physiologically acceptable salt thereof.

4. The compound according to one or more of claims 1 to 3 for use as a pharmaceutical.

5. A pharmaceutical which comprises one or more compounds according to one or more of claims 1 to 3.

6. A pharmaceutical which comprises one or more compounds according to one or more of claims 1 to 3 and at least one further active ingredient.

7. The pharmaceutical as claimed in claim 6, which comprises as further active ingredient one or more antidiabetics, hypoglycemic active ingredients, HMGCoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose-1,6-biphosphatase, modulators of glucose transporter 4, inhibitors of glutamine-fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, GPR40 modulators, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, diphenylazetidinone derivative, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

8. The use of a compound as claimed in one or more of claims 1 to 3 for manufacturing a medicament for the treatment of diabetes.

9. The use of a compound as claimed in one or more of claims 1 to 3 for manufacturing a medicament for lowering blood glucose levels.

10. The use of a compound as claimed in one or more of claims 1 to 3 for manufacturing a medicament for the treatment of metabolic syndrome.

11. The use of a compound as claimed in one or more of claims 1 to 3 for manufacturing a medicament for the treatment of nicotine addiction.

12. The use of a compound as claimed in one or more of claims 1 to 3 for manufacturing a medicament for the treatment of alcohol addiction.

13. The use of a compound as claimed in one or more of claims 1 to 3 for manufacturing a medicament for the treatment of CNS disorders.

14. The use of a compound as claimed in one or more of claims 1 to 3 for manufacturing a medicament for the treatment of schizophrenia.

15. The use of a compound as claimed in one or more of claims 1 to 3 for manufacturing a medicament for the treatment of Alzheimer's disease.

16. A method for manufacturing a pharmaceutical comprising one or more compounds according to one or more of claims 1 to 3, which comprises mixing the active ingredient with a pharmaceutically acceptable carrier, and converting this mixture into a form suitable for administration.

## Revendications

1. Composés de formule I, 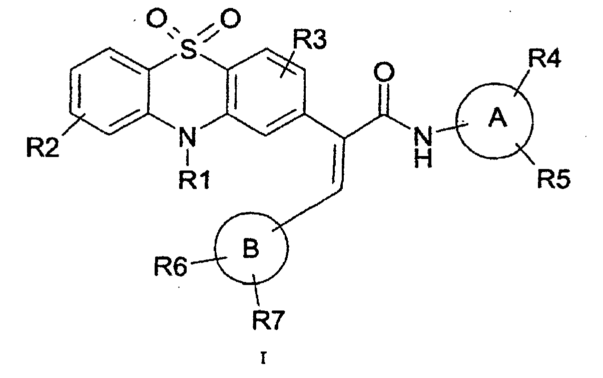 où
R1 signifie H, (C₁-C₆)-alkyle, (C₀-C₆)-alkylène-aryle, CO-(C₁-C₆)-alkyle, (C₂-C₆)-alkylène-COO-(C₀-C₆)- alkyle, (C₂-C₆)-alkylène-O-(C₁-C₆)-alkyle;
R2, R3 signifient indépendamment l'un de l'autre H, F, Cl, Br, CN, NO₂, (C₀-C₆)-alkylène-COO-(C₀-C₆)- alkyle, (C₀-C₆)-alkylène-O-(C₀-C₆)-alkyle, (C₁-C₆)-alkyle, (C₀-C₆)-alkylène-CO-(C₁-C₆)-alkyle, (C₀-C₆)-alkylène-phényle, SCF₃, SF₅, SCH₃ ;
R4, R5 signifient indépendamment l'un de l'autre H, F, Cl, Br, CN, SCN, NO₂, (C₀-C₆)-alkylène-COO-(C₀-C₆)- alkyle, -CO-COO-(C₀-C₆)-alkyle, (C₀-C₆)-alkylène-O- (C₀-C₆)-alkyle, (C₁-C₆)-alkyle, (C₀-C₆)-alkylène-CO- (C₁-C₆)-alkyle, (C₀-C₆)-alkylène-CONH(C₀-C₆)-alkyle, (C₀-C₆)-alkylène-CON[(C₀-C₆)-alkyle]₂, (C₀-C₆)- alkylène-NH(C₀-C₆)-alkyle, (C₀-C₆)-alkylène-NH-COO- (C₀-C₆)-alkyle, (C₀-C₆)-alkylène-CON[(C₀-C₆)-alkyl]- O-(C₀-C₆)-alkyle, (C₀-C₆)-alkylène-N[(C₀-C₆)- alkyle]₂, (C₀-C₆)-alkylène-aryle, SF₅, (C₀-C₆)- alkyle-S(O)ₓ(C₁-C₆)-alkyle, S(O)ₓ(C₁-C₆)-alkylène- COO- (C₀-C₆)-alkyle, S(O)ₓ(C₂-C₆)-alkylène-O-(C₀-C₆)- alkyle, -SO₂-NH- (C₀-C₆)-alkyle, -SO₂-N-[(C₀-C₆)- alkyle]₂, S(O)ₓ(C₀-C₆)-alkylène-hétérocycle, S(O)ₓ(C₁-C₆)-alkylène-CO-hétérocycle, -NH-SO₂-(C₁- C₆)-alkyle, (C₀-C₆)-alkylène-cycloalkyle, (C₀-C₆)- alkylène-hétérocycle, (C₀-C₆)-alkylène-aryle ;
R6, R7 signifient, indépendamment l'un de l'autre, H, F, Cl, Br, CN, NO₂, =O, =S, =N-O-(C₀-C₆)-alkyle, (C₀-C₆) -alkylène-COO-(C₀-C₆)-alkyle, (C₀-C₆)- alkylène-O-(C₀-C₆)-alkyle, (C₀-C₆)-alkylène-O-CO- (C₁-C₆)-alkyle, (C₁-C₆)-alkyle, (C₀-C₆)-alkylène-CO- (C₁-C₆)-alkyle, (C₀-C₆)-alkylène-aryle, SF₅, S(O)ₓ- (C₁-C₆)-alkyle ;
A signifie un hétérocycle de 5 à 10 chaînons, où l'hétérocycle peut être condensé avec un autre cycle de 5 à 10 chaînons ;
B signifie un cycle cycloalkyle de 4 à 8 chaînons, un hétérocycle de 4 à 10 chaînons ou un cycle aryle de 6 à 10 chaînons ;
où on entend par la définition -(C₀-C₆)-alkyle à chaque fois que soit un hydrogène, soit un groupe (C₁-C₆)-alkyle peut être présent,
ainsi que leurs sels physiologiquement acceptables.

2. Composés de formule I, selon la revendication 1, **caractérisés en ce que**
R1 signifie H, (C₁-C₆)-alkyle, (C₀-C₆)-alkylène-aryle, CO-(C₁-C₆)-alkyle, (C₂-C₆)-alkylène-COO-(C₀-C₆)- alkyle, (C₂-C₆)-alkylène-O-(C₁-C₆)-alkyle ;
R2, R3 signifient, indépendamment l'un de l'autre H, F, Cl, Br, CN, NO₂, (C₀-C₆)-alkylène-COO-(C₀-C₆)- alkyle, (C₀-C₆)-alkylène-O-(C₀-C₆)-alkyle, (C₁-C₆)- alkyle, (C₀-C₆)-alkylène-CO-(C₁-C₆)-alkyle, (C₀-C₆)- alkylène-phényle, SCF₃, SF₅, SCH₃ ;
R4, R5 signifient indépendamment l'un de l'autre H, F, Cl, Br, CN, SCN, NO₂, (C₀-C₆)-alkylène-COO-(C₀-C₆)- alkyle, -CO-COO-(C₀-C₆)-alkyle, (C₀-C₆)-alkylène-O- (C₀-C₆)-alkyle, (C₁-C₆)-alkyle, (C₀-C₆) -alkylène-CO- (C₁-C₆)-alkyle, (C₀-C₆)-alkylène-CONH(C₀-C₆)-alkyle, (C₀-C₆)-alkylène-CON[(C₀-C₆)-alkyle]₂, (C₀-C₆)- alkylène-NH (C₀-C₆)-alkyle, (C₀-C₆)-alkylène-NH-COO- (C₀-C₆)-alkyle, (C₀-C₆)-alkylène-CON[(C₀-C₆)-alkyl]- O-(C₀-C₆)-alkyle, (C₀-C₆)-alkylène-N[(C₀-C₆)- alkyle]₂, (C₀-C₆)-alkylène-aryle, SF₅, (C₀-C₆)- alkyle-S(O)ₓ(C₁-C₆)-alkyle, S(O)ₓ(C₁-C₆)-alkylène- COO-(C₀-C₆)-alkyle, S(O)ₓ(C₂-C₆)-alkylène-O-(C₀-C₆)- alkyle, -SO₂-NH-(C₀-C₆)-alkyle, -SO₂-N-[(C₀-C₆)- alkyle]₂, S(O)ₓ(C₀-C₆)-alkylène-hétérocycle, S(O)ₓ(C₁-C₆)-alkylène-CO-hétérocycle, -NH-SO₂-(C₁- C₆)-alkyle, (C₀-C₆)-alkylène-cycloalkyle, (C₀-C₆)- alkylène-hétérocycle, (C₀-C₆)-alkylène-aryle ;
R6, R7 signifient, indépendamment l'un de l'autre, H, F, Cl, Br, CN, NO₂, =O, =S, =N-O-(C₀-C₆)-alkyle, (C₀-C₆)-alkylène-COO-(C₀-C₆)-alkyle, (C₀-C₆)- alkylène-O-(C₀-C₆)-alkyle, (C₀-C₆)-alkylène-O-CO- (C₁-C₆)-alkyle, (C₁-C₆)-alkyle, (C₀-C₆)-alkylène-CO- (C₁-C₆)-alkyle, (C₀-C₆)-alkylène-aryle, SF₅, S (O)ₓ- (C₁-C₆)-alkyle ;
A signifie un hétérocycle de 5 à 10 chaînons, où l'hétérocycle peut être condensé avec un autre cycle de 5 à 10 chaînons ;
B signifie un cycle cycloalkyle de 4 à 8 chaînons ;
ainsi que leurs sels physiologiquement acceptables.

3. Composés de formule I, selon la revendication 1 ou 2, **caractérisés en ce que**
R1 signifie H, (C₁-C₆)-alkyle, (C₀-C₆)-alkylène-aryle, CO- (C₁-C₆)-alkyle, (C₂-C₆)-alkylène-COO-(C₀-C₆)- alkyle, (C₂-C₆)-alkylène-O-(C₁-C₆)-alkyle ;
R2, R3 signifient H ;
R4 signifie (C₁-C₆)-alkyle, O-(C₁-C₆)-alkyle, (C₀-C₆)- alkylène-aryle ;
R5 signifie H ;
R6, R7 signifient, indépendamment l'un de l'autre, H, F, Cl, Br, CN, NO₂, =O, =S, =N-O-(C₀-C₆)-alkyle, (C₀-C₆)-alkylène-COO-(C₀-C₆)-alkyle, (C₀-C₆)- alkylène-O-(C₀-C₆)-alkyle, (C₀-C₆)-alkylène-O-CO- (C₁-C₆)-alkyle, (C₁-C₆)-alkyle, (C₀-C₆)-alkylène-CO- (C₁-C₆)-alkyle, (C₀-C₆)-alkylène-aryle, SF₅, S(O)ₓ- (C₁-C₆)-alkyle ;
A signifie un hétérocycle de 5 à 10 chaînons, où l'hétérocycle peut être condensé avec un autre cycle de 5 à 10 chaînons ;
B signifie un cycle cycloalkyle de 4 à 8 chaînons ;
ainsi que leurs sels physiologiquement acceptables.

4. Composés selon l'une ou plusieurs des revendications 1 à 3 destinés à être utilisés comme médicament.

5. Médicament contenant un ou plusieurs des composés selon l'une ou plusieurs des revendications 1 à 3.

6. Médicament contenant un ou plusieurs des composés selon l'une ou plusieurs des revendications 1 à 3 et au moins une autre substance active.

7. Médicament selon la revendication 6, **caractérisé en ce qu'**il contient comme autre substance active un(e) ou plusieurs antidiabétiques, substances actives hypoglycémiques, inhibiteurs de la HMGCoA-réductase, inhibiteurs de la résorption de cholestérol, agonistes de PPAR gamma, agonistes de PPAR alpha, agonistes de PPAR alpha/gamma, agonistes de PPAR delta, fibrates, inhibiteurs de MTP, inhibiteurs de la résorption de l'acide biliaire, inhibiteurs de CETP, adsorbants polymères de l'acide biliaire, inducteurs de récepteurs de LDL, inhibiteurs d'ACAT, antioxydants, inhibiteurs de la lipoprotéine-lipase, inhibiteurs de l'ATP-citrate lyase, inhibiteurs de la squalène-synthétase, antagonistes de la lipoprotéine (a), agonistes du récepteur de HM74A, inhibiteurs de lipase, insulines, sulfonylurées, biguanides, méglitinides, thiazolidinediones, inhibiteurs de l'a-glucosidase, substances actives agissant sur le canal potassique dépendant d'ATP des cellules bêta, inhibiteurs de la glycogène phosphorylase, antagonistes du récepteur du glucagon, activateurs de la glucokinase, inhibiteurs de la gluconéogenèse, inhibiteurs de la fructose-1,6-biphosphatase, modulateurs du transporteur-4 du glucose, inhibiteurs de la glutamine-fructose-6-phosphate-amidotransférase, inhibiteurs de la dipeptidylpeptidase-IV, inhibiteurs de la 11-bêta-hydroxystéroïde-déshydrogénase-1, inhibiteurs de la protéine-tyrosine-phosphatase-1B, modulateurs du transporteur de glucose 1 ou 2 dépendant du sodium, modulateurs de GPR40, inhibiteurs de la lipase sensible aux hormones, inhibiteurs de l'acétyl-CoA carboxylase, inhibiteurs de la phosphoénol-pyruvate-carboxykinase, inhibiteurs de la glycogène synthase kinase-3 bêta, inhibiteurs de la protéine kinase C bêta, antagonistes du récepteur de l'endothéline A, inhibiteurs de la I kappaB kinase, modulateurs du récepteur des glucocorticoïdes, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes de ß3, antagonistes du récepteur de CB1, agonistes de MSH (hormone stimulant les mélanocytes), agonistes de CCK, inhibiteurs de la résorption de la sérotonine, composés sérotoninergiques et noradrénergiques mixtes, agonistes de 5HT, agonistes de bombésine, agonistes de galanine, hormones de croissance, composés libérant l'hormone de croissance, dérivés de diphénylazétidinone, agonistes de TRH, modulateurs 2 ou 3 de la protéine découplante, agonistes de leptine, agonistes de DA (Bromocriptine, Doprexine), inhibiteurs de lipase/amylase, modulateurs de PPAR, modulateurs de RXR, agonistes de TR-ß ou amphétamines.

8. Utilisation des composés selon l'une ou plusieurs des revendications 1 à 3 pour la préparation d'un médicament destiné au traitement du diabète.

9. Utilisation des composés selon l'une ou plusieurs des revendications 1 à 3 pour la préparation d'un médicament destiné à abaisser la glycémie.

10. Utilisation des composés selon l'une ou plusieurs des revendications 1 à 3 pour la préparation d'un médicament destiné au traitement du syndrome métabolique.

11. Utilisation des composés selon l'une ou plusieurs des revendications 1 à 3 pour la préparation d'un médicament destiné au traitement de la dépendance de la nicotine.

12. Utilisation des composés selon l'une ou plusieurs des revendications 1 à 3 pour la préparation d'un médicament destiné au traitement de la dépendance de l'alcool.

13. Utilisation des composés selon l'une ou plusieurs des revendications 1 à 3 pour la préparation d'un médicament destiné au traitement de maladies du système nerveux central.

14. Utilisation des composés selon l'une ou plusieurs des revendications 1 à 3 pour la préparation d'un médicament destiné au traitement de la schizophrénie.

15. Utilisation des composés selon l'une ou plusieurs des revendications 1 à 3 pour la préparation d'un médicament destiné au traitement de la maladie d'Alzheimer.

16. Procédé pour la préparation d'un médicament contenant un ou plusieurs des composés selon l'une ou plusieurs des revendications 1 à 3, **caractérisé en ce que** la substance active est mélangée avec un support pharmaceutiquement approprié et ce mélange est amené dans une forme appropriée pour l'administration.
